# EUROPEAN PATENT SPECIFICATION

(11) **EP 3 116 901 B1**
(45) Date of publication and mention of the grant of the patent: **12.06.2019**
(21) Application number: 15709505.0
(22) Date of filing: 13.03.2015
(51) Int. Cl.: C40B 40/10, C40B 40/02

(54) **TCR LIBRARIES**
TCR-BIBLIOTHEKEN
BANQUES DE TCR

(30) Priority: 14.03.2014 GB 201404536; 14.03.2014 US 201461953114 P; 02.12.2014 GB 201421336
(43) Date of publication of application: 18.01.2017
(73) Proprietor: Immunocore Limited, Abingdon, Oxfordshire OX14 4RY (GB); Adaptimmune Limited, Oxford Business Park Oxford OX2 2HN (GB)
(72) Inventor: JAKOBSEN, Bent Karsten, Abingdon Oxfordshire OX14 4RY (GB); MOLLOY, Peter Eamon, Abingdon Oxfordshire OX14 4RY (GB); VUIDEPOT, Annelise Brigitte, Abingdon Oxfordshire OX14 4RY (GB); LIDDY, Nathaniel Ross, Abingdon Oxfordshire OX14 4RY (GB)
(74) Representative: Care, Alison
(86) International application number: PCT/EP2015/055293
(87) International publication number: WO 2015/136072

(56) References cited:
- WO-A2-2005/116074
- TAKAJI MATSUTANI ET AL: "Analysis of TCRAV and TCRBV Repertoires in Healthy Individuals by Microplate Hybridization Assay", HUMAN IMMUNOLOGY, vol. 56, no. 1-2, 1 August 1997 (1997-08-01), pages 57-69, XP055191940, ISSN: 0198-8859, DOI: 10.1016/S0198-8859(97)00102-X
- DUNN STEVEN M ET AL: "Directed evolution of human T cell receptor CDR2 residues by phage display dramatically enhances affinity for cognate peptide-MHC without increasing apparent cross-reactivity", PROTEIN SCIENCE, WILEY, US, vol. 15, no. 4, 1 April 2006 (2006-04-01), pages 710-721, XP002465974, ISSN: 0961-8368, DOI: 10.1110/PS.051936406

## Description

### Summary of the Invention

The present invention relates to a library of particles, the library displaying a plurality of different T cell receptors (TCRs), wherein the plurality of TCRs consists essentially of TCRs comprising an alpha chain variable domain from a natural repertoire and a beta chain variable domain from a natural repertoire, wherein the alpha chain variable domain comprises a TRAV12-2 or a TRAV21 gene product and the beta chain variable domain comprises a TRBV6 gene product.

### Background

T cell receptors (TCRs) mediate the recognition of specific major histocompatibility complex (MHC)-restricted peptide antigens by T cells and are essential to the functioning of the cellular arm of the immune system. TCRs exist only in membrane bound form and for this reason TCRs are historically very difficult to isolate. Most TCRs are composed of two disulphide linked polypeptide chains, the alpha and beta chain.

TCRs are described herein using the International Immunogenetics (IMGT) TCR nomenclature and links to the IMGT public database of TCR sequences. Native alpha-beta heterodimeric TCRs have an alpha chain and a beta chain. Broadly, each chain comprises variable, joining and constant regions, and the beta chain also usually contains a short diversity region between the variable and joining regions, but this diversity region is often considered as part of the joining region. Each variable region comprises three hypervariable CDRs (Complementarity Determining Regions) embedded in a framework sequence; CDR3 is believed to be the main mediator of antigen recognition. There are several types of alpha chain variable (Vα) regions and several types of beta chain variable (Vβ) regions distinguished by their framework, CDR1 and CDR2 sequences, and by a partly defined CDR3 sequence. The Vα types are referred to in IMGT nomenclature by a unique TRAV number. Thus "TRAV21" defines a TCR Vα region having unique framework and CDR1 and CDR2 sequences, and a CDR3 sequence which is partly defined by an amino acid sequence which is preserved from TCR to TCR but which also includes an amino acid sequence which varies from TCR to TCR. In the same way, "TRBV6-5" defines a TCR Vβ region having unique framework and CDR1 and CDR2 sequences, but with only a partly defined CDR3 sequence. For the purposes of this application, we are using the general assumption that there are 54 functional alpha variable genes and 67 functional beta variable genes within the alpha and beta loci respectively. However, this number may vary as research progresses and the number of alpha and beta variable genes may be considered to be different than at the present time due to definitions of functionality or duplications. Thus, for the sake of clarity, we consistently refer to the International Immunogenetics (IMGT) TCR nomenclature as found at the IMGT website www.imgt.org (as accessed 10 March 2013).

The joining regions of the TCR are similarly defined by the unique IMGT TRAJ and TRBJ nomenclature, and the constant regions by the IMGT TRAC and TRBC nomenclature.

The beta chain diversity region is referred to in IMGT nomenclature by the abbreviation TRBD, and, as mentioned, the concatenated TRBD/TRBJ regions are often considered together as the joining region.

The gene pools that encode the TCR alpha and beta chains are located on different chromosomes and contain separate V, (D), J and C gene segments, which are brought together by rearrangement during T cell development. This leads to a very high diversity of T cell alpha and beta chains due to the large number of potential recombination events that occur between the 54 TCR alpha variable genes and 61 alpha J genes or between the 67 beta variable genes, two beta D genes and 13 beta J genes. The recombination process is not precise and introduces further diversity within the CDR3 region. Each alpha and beta variable gene may also comprise allelic variants, designated in IMGT nomenclature as TRAVxx*01 and *02, or TRBVx-x*01 and *02 respectively, thus further increasing the amount of variation. In the same way, some of the TRBJ sequences have two known variations. (Note that the absence of a"*" qualifier means that only one allele is known for the relevant sequence.). The natural repertoire of human TCRs resulting from recombination and thymic selection has been estimated to comprise approximately 10⁶ unique beta chain sequences, determined from CDR3 diversity (Arstila, T. P., et al (1999) Science, 286(5441), 958-61*)* and could be even higher *(*Robins, H.S. et al. (2009) Blood, 114(9), 4099-4107*).* Each beta chain is estimated to pair with at least 25 different alpha chains thus generating further diversity (Arstila, T. P., et al (1999) Science, 286(5441), 958-61*).*

In the present specification and claims, the term "TCR alpha (or α) variable domain" therefore refers to the concatenation of TRAV and TRAJ regions; a TRAV region only; or TRAV and a partial TRAJ region, and the term TCR alpha (or α) constant domain refers to the extracellular TRAC region, or to a C-terminal truncated TRAC sequence. Likewise the term "TCR beta (or β) variable domain" may refer to the concatenation of TRBV and TRBD/TRBJ regions; to the TRBV and TRBD regions only; to the TRBV and TRBJ regions only; or to the TRBV and partial TRBD and/or TRBJ regions, and the term TCR beta (or β) constant domain refers to the extracellular TRBC region, or to a C-terminal truncated TRBC sequence.

The unique sequences defined by the IMGT nomenclature are widely known and accessible to those working in the TCR field. For example, they can be found in the IMGT public database. The "T cell Receptor Factsbook", (2001) LeFranc and LeFranc, Academic Press, ISBN 0-12-441352-8 also discloses sequences defined by the IMGT nomenclature, but because of its publication date and consequent time-lag, the information therein sometimes needs to be confirmed by reference to the IMGT database.

It has long been desirable to identify TCRs consisting essentially of natural alpha and beta chain sequences that specifically bind to particular antigens, such that for example the TCRs, or their soluble analogues, can be developed to provide basis for potential therapeutics. The antigens recognised by the identified TCRs may be associated with a disease, such as cancer, viral infections, autoimmune diseases, parasitic infections and bacterial infections. Therefore, such therapies can be used for the treatment of said diseases.

Furthermore, once natural or native TCRs have been identified and their sequences determined, mutations can be introduced that result in an increase in affinity or half-life, as needed, such as described in WO2012/013913.

Traditionally, attempts to identify TCRs that specifically bind to disease-associated antigens, such as cancer viral, autoimmune or bacterialantigens, have been limited to the use of blood samples taken from volunteer donors. Such samples are used to isolate T cells and their corresponding TCRs which bind disease associated antigens. This approach generally requires at least 20 donors. The process is long and labour intensive, and there is no guarantee of identifying antigen binding T cell receptors. Where functional T cell receptors are identified they often have weak affinity for antigen, low specificity, and/or do not fold properly *in vitro.* The diversity of T cells that are able to be screened is limited to the T cell diversity within donors. Some disease-associated antigens, including the majority of cancer-antigens, are self-antigens; since thymic selection serves to remove TCRs that recognise self-antigens, TCRs specific for disease associated antigens may not be present in the natural repertoire of the donors, or else may have weak affinity for antigen.

Attempts to design a library for the isolation of new TCRs with antigen binding specificity have been on-going for several years. TCRs libraries are far more difficult to create than comparable antibody libraries, since TCR chains are less stable and often do not display correctly. The complexities involved in constructing a library of TCRs are enormous. Retaining variation in CDR3 length, (as found in natural repertoires) is preferable. A substantial portion of any library is generally lost to stop codons, frame shifts, folding problems and TCR chain combinations that could simply never bind to an HLA complex. Taking into account the huge number of variable alpha and variable beta genes, as well as the J and D genes, the chance of producing and identifying a functional folding alpha chain and a functional folding beta chain that together form a TCR that binds to an antigenic peptide with the required specificity, is extremely low.

A number of attempts at constructing libraries have been made. The first herein described below are based on synthetic TCR libraries; that is, the TCRs in the library contain mutations, typically within the CDRs, which have been introduced *in vitro* using random mutagenesis. Therefore, the sequences of any individual TCR chain contained in these libraries may not correspond to any found in a natural repertoire. The whole library will not correspond to a natural repertoire due to only certain mutations being present in the synthetic libraries. In the previously disclosed synthetic libraries random mutations were introduced into the CDR regions of alpha and beta chains of a single known TCR, such that all TCRs in the library contain the same alpha and beta framework sequence but with randomly generated CDR sequences. Further analysis of the library demonstrated that it was not successful for the identification of antigen specific TCRs. Specifically, it was found that a large proportion of the TCR chains were non-functional, for various reasons: in many cases the sequences were truncated or contained frameshifts. In other cases, although full length TCR chains were identified they were unable to fold correctly; finally, TCRs isolated from the library were not able to specifically bind an antigen when subjected to further testing. It is thought that the non-natural diversity in these synthetic libraries may be one reason why the libraries were not successful. The introduction of non-natural mutations may interfere with proper TCR function. Furthermore, the introduced diversity in CDR3 may be limited compared to a natural TCR repertoire. As exemplified by CDR3 sequence length in a natural repertoire, a huge diversity in CDR3 sequences is generated during TCR assembly in T cells. By basing a library on mutations at specific locations, the diversity of CDR3 sequences may be very much restricted, particularly in respect of the CDR3 sequence length. Finally, synthetic TCR sequences will not have been subjected to the thymic selection process that occurs *in vivo.*

These reasons go some way to explain, without wishing to be bound by theory, why the attempts to build libraries from which specifically binding TCRs were hoped to be identified, described below, were not successful.

WO2005/116646 describes a library based on a known (natural) TCR in which the six CDRs were mutated individually or in combination, i.e. all TCRs in the library were synthetic but based on a naturally identified TCR framework region. WO 2005/114215 further relates to products obtained from such a library. The library was screened with several other antigens (in addition to that to which the original TCR bound). However, this resulted in only one productive full-length TCR sequence being isolated. In further experiments, it was found that this TCR was cross reactive.

Thus, libraries based on *in vitro*-mutated TCRs have been constructed, but have not enabled the isolation of new TCRs with antigen binding specificity.

A library based on an entirely natural repertoire wherein the naturally derived alpha and beta chains were mixed randomly, (as discussed below), has been constructed but was not successful in identifying any TCRs which specifically bind antigen.

In particular, WO2005/116074 describes a library of nucleoproteins, each displaying on its surface a polypeptide comprising a native TCR alpha variable domain sequence or a native TCR beta variable domain sequence. The library described in this publication was constructed from a number of alpha and beta chains; 43 V alpha class genes and 37 V beta class genes were represented in the mRNA pool from which the cDNA used to generate the library was amplified. It is stated in this document that three rounds of phage display led to the isolation of clones which bound to the peptide being tested. These clones are described as having been identified during ELISA screening as determined by strong ELISA signals. However, strong ELISA signals were also observed when these clones were tested for binding an alternative peptide-HLA; therefore, the TCR clones were not specific for peptide. Further analysis of this library indicated similar issues to those described above for synthetic libraries in that they contained a large proportion of non-productive TCR chains as well as TCRs that were unable to fold correctly. The library described therein was thus not useful for identifying new antigen-binding TCRs.

Therefore, there is need for a TCR library that enables the more reliable identification of functional TCRs comprising a natural alpha chain variable domain and a natural beta chain variable domain, which library may be screened using a variety of peptide antigens in order to identify such useful TCRs. The identified TCRs can then either be used at their natural affinity or could be used in, for example, phage display maturation, to enhance affinity.

Therefore, the present invention provides in a first aspect, a library of particles, the library displaying a plurality of different T cell receptors (TCRs), wherein the plurality of TCRs consists essentially of TCRs comprising an alpha chain comprising an alpha chain variable domain from a natural repertoire and a beta chain comprising a beta chain variable domain from a natural repertoire, wherein the alpha chain variable domain comprises a TRAV12-2 or a TRAV21 gene product and the beta chain variable domain comprises a TRBV6 gene product. Variable domains are as delivered above i.e. they may also comprise complete or partial TRAJ or TRBD and/or TRBJ regions, respectively.

The invention also provides as a second aspect a library of particles, the library displaying a plurality of different TCRs, wherein the plurality of TCRs consists essentially of TCRs comprising an alpha chain comprising an alpha chain variable domain from a natural repertoire and a beta chain comprising a beta chain variable domain from a natural repertoire, wherein the alpha chain variable domain comprises a TRAV12-2 or a TRAV21 gene product and the beta chain variable domain comprises a TRBV6 gene product and wherein at least a portion of the TCRs comprise an alpha chain variable domain and/or a beta chain variable domain comprising a non-natural mutation.

The TRBV6 gene product may be any of a TRBV6-1, a TRBV6-2, a TRBV6-3, a TRBV6-5 or a TRBV6-6 gene product. The TCR alpha chain variable domain comprises a TRAV12-2 gene product. Alternatively, the TCR alpha chain variable domain comprises a TRAV21 gene product.

The alpha chain variable domain and the beta chain variable domain may be displayed as a single polypeptide chain.

The TCRs are displayed on particles and may comprise a native or non-native disulphide bond between a constant region of the alpha chain and a constant region of the beta chain. Such non-native di-sulphide bonds are described for example, in WO 03/020763. When a TCR displayed on a particle of the library comprises a constant region 1 domain, the TCRs may comprise a native disulphide bond between a constant region of the alpha chain and a constant region of the beta chain.

Each alpha chain and each beta chain may comprise a dimerization domain, which is preferably heterologous. Such a heterologous domain may be a leucine zipper, a 5H3 domain or hydrophobic proline rich counter domains, or other similar modalities, as known in the art.

The particles forming the library may be phage particles.

Alternatively, the library may be a library of ribosomes. Alternatively, the library may be a yeast display library, so the particles may be yeast cells. Alternatively, the library may be a library of mammalian cells.

A further aspect of the invention provides a non-natural isolated T cell receptor (TCR) comprising a TCR alpha chain variable domain comprising a TRAV12-2 gene product or a TRAV21 gene product and a TCR beta chain variable domain comprising a TRBV6 gene product obtained from a library of the second aspect of the invention.

The TRBV6 gene product of such a TCR may be a TRBV6-1, a TRBV6-2, a TRBV6-3, a TRBV6-5 or a TRBV6-6 gene product. The TCR is preferably soluble. Also encompassed by the invention is a nucleic acid encoding a TCR alpha chain variable domain and/or a beta chain variable domain of the TCR.

As a further aspect, the invention provides the use of a library of the first or second aspect, to identify a TCR that specifically binds to a peptide antigen. The peptide antigen may be used to screen the library of the invention for a TCR to which it binds.

In a further aspect, the invention provides a method of obtaining a T cell receptor that specifically binds a peptide antigen, comprising screening the library of the first or second aspects with the peptide antigen, the method comprising: a) panning the library using as a target the peptide antigen; repeating step a) one or more times; c) screening the phage clones identified in step a) or b); and d) identifying a TCR that specifically binds the peptide antigen.

In a further aspect, the invention is concerned with a method of making a library of particles, the library displaying a plurality of different TCRs, the method comprising: i) obtaining a plurality of nucleic acids from a natural repertoire that encode different TRAV12-2 or TRAV21 alpha chain variable domains; ii) obtaining a plurality of nucleic acids from a natural repertoire that encode different TRBV6 beta chain variable domains; iii) cloning the TRAV12-2 or TRAV21 alpha chain variable domain encoding nucleic acids into expression vectors; iv) cloning the TRBV6 beta chain variable domain encoding nucleic acids into the same or different vectors; and v) expressing the vectors in particles, thereby generating a library consisting essentially of TCRs comprising an alpha chain variable domain and a beta chain variable domain encoded by the nucleic acids.

A further method of the invention of making a library of particles is provided, the library displaying a plurality of different TCRs, the method comprising: i) obtaining a plurality of nucleic acids from a natural repertoire that encode different TRAV12-2 or TRAV21 alpha chain variable domains using primers that hybridise to nucleic acids encoding TRA12-2 or TRAV21 alpha chain variable domains; ii) obtaining a plurality of nucleic acids from a natural repertoire that encode different TRBV6 beta chain variable domains using primers that hybridise to nucleic acids encoding TRAV6 beta chain variable domains iii) cloning the TRAV12-2 or TRAV21 alpha chain variable domain encoding nucleic acids into expression vectors; iv) cloning the TRBV6 beta chain variable domain encoding nucleic acids into the same or different vectors; and v) expressing the vectors in particles, thereby generating a library consisting essentially of TCRs comprising an alpha chain variable domain and a beta chain variable domain encoded by the nucleic acids to which said primers hybridise.

A forward primer may be designed to hybridise to the TRAV12-2 locus, the TRAV21 locus or the TRBV6 locus. A reverse primer may be designed to hybridise, at least in part to the alpha or beta constant region, respectively, such that the resulting PCR product contains the variable regions, through to the joining regions and at least part of the constant region. Transcription, translation or post-translation events may result in truncation, or deletion of some or all of the joining and/or constant regions, including the diversity region in the case of the beta chain sequences.

In some instances, non-natural mutations may be introduced to the nucleic acids prior to step iii). The mutations may be introduced after step i) and/or ii)

The TRBV6 beta china variable domains may be TRBV6-1, TRBV6-2, TRBV6-3, TRBV6-5 or TRBV6-6 beta chain variable domains.

In either method of making a library of the invention the TCR alpha chain variable domain and the TCR beta chain variable domain are preferably expressed as a single chain polypeptide, i.e. nucleic acids that encode each of the alpha and beta chain variable domains are cloned into the same vector.

The invention provides as a further aspect a method of obtaining a T cell receptor that specifically binds a peptide antigen, comprising screening a library of the first or second aspects of the invention with the peptide antigen.

A particle displaying on its surface a TCR in accordance with the invention is also included in the scope of the present invention. The particle may be a phage, a ribosome, a yeast cell or a mammalian cell.

The library of the invention is non-naturally occurring as it includes TCR(s) that are not naturally occurring or those that would be considered "isolated" as that term is used herein; and accordingly, TCRs of the invention are likewise patent-eligible subject matter as such TCRs are not naturally occurring or those that would be considered "isolated" as that term is used herein. Similarly, cells and particles of the invention are patent-eligible subject matter because by displaying on its surface or expressing a TCR of the invention, the cell or particle is not naturally occurring or that which would be considered "isolated" as that term is used herein.

It is also noted that in this disclosure and particularly in the claims and/or paragraphs, terms such as "comprises", "comprised", "comprising" and the like can have the usual meaning attributed to it; e.g., they can mean "includes", "included", "including", and the like; and that terms such as "consisting essentially of' and "consists essentially of' have the meaning generally ascribed to them e.g., they allow for elements not explicitly recited, but exclude elements that are found in the prior art or that affect a basic or novel characteristic of the invention.

These and other embodiments are disclosed or are obvious from and encompassed by, the following description.

### Brief description of the drawings

The following detailed description, given by way of example, but not intended to limit the invention solely to the specific embodiments described, may best be understood in conjunction with the accompanying drawings, wherein:
Figure 1 outlines the cloning strategy used for library creation;
Figure 2 details the primer sequences used in the library creation;
Figure 3 shows results from ELISA screening of a pooled TRAV12.2/21 TRBV 6* library panned with 4 different peptide HLA antigens. CMV indicates negative control antigen;
Figure 4 shows results from ELISA screening of a pooled TRAV12.2/21 TRBV 6* library prepared from a single HLA-A2/A24 negative donor and panned with 3 peptide HLA antigens. CMV indicates negative control antigen;
Figure 5 shows results from ELISA screening of individual TRAV12.2 TRBV 6* / TRAV21 TRBV 6* libraries panned with 2 peptide HLA antigens. CMV indicates negative control antigen;
Figure 6 shows results from ELISA screening of individual TRAV12.2 TRBV 6* / TRAV21 TRBV 6* libraries prepared from a commercial mRNA source and panned with 2 peptide HLA antigens. CMV indicates negative control antigen;
Figure 7 shows further specificity testing of TCRs isolated from a library of the invention; and
Figure 8 shows Biacore binding curves for soluble versions of antigen specific TCRs isolated from a library of the invention.

According to the invention, there is provided a library of particles, the library displaying a plurality of different T cell receptors (TCRs), wherein the plurality of TCRs consists essentially of TCRs comprising an alpha chain comprising an alpha chain variable domain from a natural repertoire and a beta chain comprising a beta chain variable domain from a natural repertoire wherein the alpha chain variable domain comprises a TRAV12-2 or a TRAV21 gene product and the beta chain variable domain comprises a TRBV6 gene product.

By "consisting essentially of" it is meant that the majority of the TCRs in the library comprise TRAV12-2 or TRAV21 and TRBV6 but that the minority may comprise different alpha or beta chain variable domains due to non specific hybridisation of primers when making the library, or regions of high homology between genes in the alpha or beta variable loci genes. The amount of the majority may be defined as below.

The plurality of TCRs may consist of 80% of TCRs comprising an alpha chain variable domain comprising a TRAV12-2 or TRAV21 gene product and a beta chain variable domain comprising a TRBV6 gene product. The plurality of TCRs may consist of 85%, 90%, 91%, 92%, 93%, 94%, 95%, 96%, 97%, 98%, 99%, 100% of TCRs comprising an alpha chain variable domain comprising a TRAV12-2 or TRAV21 gene product and a beta chain variable domain comprising a TRBV6 gene product.

The remaining 20% or less of the plurality of TCRs may comprise different alpha chain variable domain gene products paired with TRBV6 beta chain variable domain gene products, different beta chain variable domain gene products paired with TRAV12-2 or TRAV21 variable domain gene products or truncated/non-productive chains.

Thus, in some embodiments, the TCR alpha chain variable domain comprises a TRAV12-2 gene product and in other embodiments, the TCR alpha chain variable domain comprises a TRAV21 gene product. The library may include a plurality of TCRs wherein a portion of the TCRs comprise a TRAV12-2 gene product and a portion of the TCRs comprise a TRAV21 gene product.

The library of the present invention may therefore contain a plurality of TCRs each having the following alpha chain and beta chain V, J, (D) and C gene usage:
alpha chain - TRAV21/TRAJxx/TRAC; or
alpha chain - TRAV12-2/TRAJxx/TRAC; and
beta chain - TRBV6-y/TRBDx/TRBJxx/TRBC1, TRBC2 or a chimera of C1 and C2, wherein xx is any of the 61 alpha J genes or 13 beta J genes, respectively, and Dx represents either of the 2 beta D genes. TRBV6-y indicates that the TRBV6 allele that is used may vary.

The TRBV gene product may be a TRBV6-1, a TRBV6-2, a TRBV6-3, TRBV6-5 or a TRBV6-6 gene product.

As discussed above the J, D or C regions may each be fully or partially present or absent.

By "from a natural repertoire" it is meant that the TCR alpha and beta chain variable domains are expressed from DNA sequences that have been obtained from human donors. In other words, the diversity of the alpha and beta variable domains of the TCRs of the library has been naturally generated during T cells development *in vivo.* Furthermore, this means that the sequences of all the alpha and beta chains in the library will have been selected for during thymic selection. The random combination of these alpha and beta chains, which occurs during library creation, may result in an alternative repertoire of alpha beta chain combinations compared to that originally present *in vivo* (i.e. in the donor(s)). The DNA sequences may be obtained indirectly e.g. by producing cDNA from donor mRNA. The cDNA sequences may then be used as templates to produce DNA sequences from which the plurality of different TCRs is produced.

By gene product it is meant a polypeptide, which may include post-translation modification, that is encoded by the nucleic acid sequence of the indicated gene. As is known to the skilled person, each TCR alpha or beta chain variable domain gene contains variation in the CDR3 regions, as discussed above, meaning that the gene products will also vary enormously.

The library of the present invention preferably comprises at least 1 x 10⁸ particles that display an αβ TCR chain combination.

The library may be a library of phage particles. Phage display is described in WO 2004/044004.

Alternatively, the library is a library of ribosomes. Ribosome display is known in the art. The particles may be complete ribosomal complexes or parts thereof.

Yeast display systems may be used, meaning that the library may be a library of yeast cells.

An additional display methodology suitable for the creation of TCRs libraries is mammalian cell display. This system uses a retroviral vector to introduce the TCR alpha and beta chains into a TCR-negative T cell hybridoma. The method is further described in Chervin et al. (2008) J Immunol Methods, 339, 175-84; and Kessels et al. (2000) Proc Natl Acad Sci U S A, 97, 14578-83).

Any library of particles that is able to display heterodimeric or single chain TCRs, as described, is encompassed by the invention.

The alpha and/or beta chain constant domain may be truncated relative to the native/naturally occurring TRAV/ TRBV sequences. In addition, where present, the TRAC/ TRBC may contain modifications. The alpha chain extracellular sequence may include a modification in relation to the native/naturally occurring TRAC whereby amino acid T48 of TRAC, with reference to IMGT numbering, is replaced with C48. Likewise, the beta chain extracellular sequence may include a modification in relation to the native/naturally occurring TRBC1 or TRBC2 whereby S57 of TRBC1 or TRBC2, with reference to IMGT numbering, is replaced with C57, and C75 is replaced by A75 and N89 replaced D89. These cysteine substitutions relative to the native alpha and beta chain extracellular sequences enable the formation of a non-native interchain disulphide bond which stabilises the refolded soluble TCR, i.e. the TCR formed by refolding extracellular alpha and beta chains. This non-native disulphide bond facilitates the display of correctly folded TCRs on phage. (Li, Y., et al. Nat Biotechnol 2005: 23(3), 349-54). In addition the use of the stable disulphide linked soluble TCR enables more convenient assessment of binding affinity and binding half-life. Alternative substitutions are described in WO03/020763. Alternatively, the alpha and beta constant domains may be linked by a disulphide bond which corresponds to that found in nature.

To further, or alternatively, stabilise the heterodimeric TCRs, each alpha chain and each beta chain may comprise a dimerization domain, which may be heterologous to the native TCR chain sequence.

In particular, the dimerization domain may be a leucine zipper. This term describes pairs of helical peptides which interact with each other in a specific fashion to form a heterodimer. The interaction occurs because there are complementary hydrophobic residues along one side of each zipper peptide. The nature of the peptides is such that the formation of heterodimers is very much more favourable than the formation of homodimers of the helices. Leucine zippers may be synthetic or naturally occurring, such as those described in WO99/60120. Alternative dimerization domains include disulphide bridge-forming elements. Alternatively, it may be provided by the SH3 domains and hydrophobic/proline rich counterdomains, which are responsible for the protein-protein interactions seen among proteins involved in signal transduction (reviewed by Schlessinger, (Schlessinger, J., Curr Opin Genet Dev. 1994 Feb; 4(1):25-30). Other natural protein-protein interactions found among proteins participating in signal transduction cascades rely on associations between post-translationally modified amino acids and protein modules that specifically recognise such modified residues. Such post-translationally modified amino acids and protein modules may form the dimerisation domain of the TCR chains of the library in accordance with the invention.

Without being bound by theory, the size of the library of the present invention, i.e. the reduced number of alpha and beta chain variable domain genes that are represented therein in relation to a full (or near full) repertoire, is thought to be a possible reason why specific functional TCRs are able to be identified from the library of the invention. In the larger "natural" libraries previously described, it may be that certain alpha chains do not pair with certain beta chains, and thus much of the library is nonfunctional. It may be that certain chain types do not fold correctly on the surface of phage. It may be that each alpha chain is not expressed or displayed sufficiently frequently to be paired with the "ideal" beta chain, and vice versa, and thus reducing the chances of identifying a specific functional TCR comprising an alpha and beta chain. Furthermore, it may be that certain alpha or beta chain sequences are dominant and therefore act to 'poison' the library.

The present invention also provides a library of particles, the library displaying a plurality of different TCRs, wherein the plurality of TCRs consists essentially of TCRs comprising an alpha chain variable domain from a natural repertoire and a beta chain variable domain from a natural repertoire, wherein the alpha chain variable domain comprises a TRAV12-2 or a TRAV21 gene product and the beta chain variable domain comprises a TRBV6 gene product and wherein at least a portion of the TCRs comprise an alpha chain variable domain and/or a beta chain variable domain comprising a non-natural mutation.

Non-natural mutations may be introduced by any way known in the art. Non-natural mutations may be randomly generated, or specifically defined, or both. For example, randomly generated mutations may be incorporated at defined positions using site-saturation mutagenesis in which the native amino acid coding sequence is replaced by the coding sequence of all other naturally occurring amino acids; thereby, creating additional library diversity at a defined position. The method may involve replicating the DNA of interest using PCR amplification with degenerate synthetic oligonucleotides as primers. Alternatively, or additionally, defined mutations, including insertions and deletions, may be introduce at certain positions using, for example, commercially available kits, such as the Quik Change Site Directed Mutagensis Kit from Stratagene. Preferably, non-natural mutations are incorporated into the CDR regions.

The library may display TCRs where 10%, 15%, 20%, 25%, 30%, 40%, 50%, 60%, 70%, 75%, 80%, 85%, 90%, 95% or 100% of the alpha chain variable domains or beta chain variable domains comprise a non-natural mutation.

As a further aspect, the invention provides an isolated T cell receptor (TCR) comprising a TCR alpha chain variable domain comprising a TRAV12-2 or a TRAV21 gene product and a TCR beta chain variable domain comprising a TRBV6 gene product isolated from a library according to the second aspect of the invention.

By isolated it is meant that the TCR is removed from its natural environment, i.e. not a TCR that is displayed naturally on a T cell *in vivo.*

The TCR may specifically bind to a peptide antigen. Such a TCR obtained from the library of the invention may bind with strong affinity and high specificity to the peptide antigen, as determined by, for example but not limited to, ELISA or BiaCore. The TCR may be taken through further affinity maturation such that binding affinity and/or half-life is increased. The TCR may be soluble, i.e. it may be cleaved from the transmembrane domain, such as described in WO 03/020763. The TCR may contain a non-native disulphide bond as described above. The TCR may be fused to detectable labels including, but not limited to, fluorescent labels, radiolabels, enzymes, nucleic acid probes and contrast reagents, or to therapeutic agents including, but not limited to, immunomodulators, radioactive compounds, enzymes (perforin for example) or chemotherapeutic agents (cis-platin for example) (WO2010/133828). The TCR may be non-naturally expressed on the surface of cells, preferable mammalian cells, more preferably immune cells, even more preferable T cells.

Binding affinity (inversely proportional to the equilibrium constant K_{D}) and binding half-life (expressed as T½) can be determined by any appropriate method. It will be appreciated that doubling the affinity of a TCR results in halving the K_{D}. T½ is calculated as ln2 divided by the off-rate (k_{off}). So doubling of T½ results in a halving in k_{off}. K_{D} and k_{off} values for TCRs are usually measured for soluble forms of the TCR, i.e. those forms which are truncated to remove hydrophobic transmembrane domain residues. Therefore it is to be understood that a given TCR meets the requirement that it has a binding affinity for, and/or a binding half-life for a peptide antigen if a soluble form of that TCR meets that requirement. Preferably the binding affinity or binding half-life of a given TCR is measured several times, at a defined temperature using the same assay protocol and an average of the results is taken. More preferable the binding affinity or binding half life is measured by surface plasmon resonance at a temperature of 25°C. A preferred method is given in Example 10.

For the purposes of the present invention, as described above, a TCR is a moiety having at least one TCR alpha and at least one TCR beta variable domain. Generally it will comprise both a TCR alpha variable domain and a TCR beta variable domain. They may be αβ heterodimers or may be single chain format, by which it is meant a single polypeptide contains both the alpha chain and the beta chain, such as described in WO 2004/033685. Alternatively the TCR may comprise a TCR α chain extracellular domain dimerised to a TCR β chain extracellular domain by means of a pair of C-terminal dimerisation peptides, such as leucine zippers, such TCRs are described in WO 99/60120. For use in adoptive therapy, an αβ heterodimeric TCR may, for example, be transfected into cells, such as T cells, as full length chains having both cytoplasmic and transmembrane domains. If desired, an introduced disulphide bond between residues of the respective constant domains may be present (see for example WO 2006/000830). Alternatively, the alpha and beta constant domains may be linked by a disulphide bond which corresponds to that found in nature.

It is important to note that, whatever the format, the TCRs of the library of the first aspect of the invention are, insofar as the alpha and beta variable domains are concerned, derived from naturally occurring sequences which have not been modified or mutated with reference to the donor mRNA that serves as the template for generating the cDNA from which the TCR chain are amplified.

Included in the invention is a nucleic acid that encodes a TCR alpha chain variable domain and/or a TCR beta chain variable domain of the TCR of the invention. The alpha and beta chains may be expressed from separate nucleic acids or from one nucleic acid molecule. If from the same nucleic acid molecule, the alpha and beta chains may be expressed as independent polypeptides, or as a single chain.

The nucleic acid comprises a TRAV12-2 or TRAV21 sequence and/or a TRBV6 nucleic acid sequence. The nucleic acid may also comprise a TRAJ sequence and/or a TRBD/TRBJ sequence. The nucleic acid may also comprise the TRAC and/or TRBC1 or TRBC2 nucleic acid sequence, or partial sequences thereof.

In a further aspect of the invention, the use of the library of the first or second aspect to identify a TCR that specifically binds a peptide antigen is provided. As mentioned, TCRs that bind specifically to a peptide antigen are desirable for a variety of reasons.

A further aspect of the invention provides a method of making a library according to the first aspect of the invention. The method comprises: i) obtaining a plurality of nucleic acids from a natural repertoire that encode different TRAV12-2 or TRAV21 alpha chain variable domains; ii) obtaining a plurality of nucleic acids from a natural repertoire that encode different TRBV6 beta chain variable domains; iii) cloning the TRAV12-2 or TRAV21 alpha chain variable domain encoding nucleic acids into expression vectors; iv) cloning the TRBV6 beta chain variable domain encoding nucleic acids into the same or different vectors; and v) expressing the vectors in particles, thereby generating a library consisting essentially of TCRs comprising an alpha chain variable domain and a beta chain variable domain encoded by the nucleic acids.

The nucleic acids may be obtained by PCR, or built synthetically, for example using solid phase DNA synthesis, such as carried out commercially by Life Technologies. The nucleic acids of i) and ii) may be obtained by copying/amplifying the nucleotide sequence trans cDNA, which has been made from mRNA from a donor's T cell repertoire. The nucleic acids that are obtained that encode different TRAV12-2 or TRAV21 alpha or TRBV6 beta chain variable domains may be the only nucleic acids obtained i.e. step i) may involve obtaining only nucleic acids that encode different TRAV12-2 or TRAV21 alpha chain variable domains and step ii) may involve obtaining only nucleic acids that encode different TRBV6 beta chain variable domains. The library generated may be a library consisting essentially of TCRs comprising an alpha chain variable domain from a natural repertoire and a beta chain variable domain from a natural repertoire, wherein the alpha chain variable domain comprises a TRAV12-2 or a TRAV21 gene product and the beta chain variable domain comprises a TRBV6 gene product.

The invention also provides a method of making a library of particles, the library displaying a plurality of different TCRs, the method comprising: i) obtaining a plurality of nucleic acids from a natural repertoire that encode different TRAV12-2 or TRAV21 alpha chain variable domains using primers that hybridise to nucleic acids encoding TRA12-2 or TRAV21 alpha chain variable domains; ii) obtaining a plurality of nucleic acids from a natural repertoire that encode different TRBV6 beta chain variable domains using primers that hybridise to nucleic acids encoding TRAV6 beta chain variable domains; iii) cloning the TRAV12-2 or TRAV21 alpha chain variable domain encoding nucleic acids into expression vectors; iv) cloning the TRBV6 beta chain variable domain encoding nucleic acids into the same or different vectors; and v) expressing the vectors in particles, thereby generating a library consisting essentially of TCRs comprising an alpha chain variable domain and a beta chain variable domain encoded by the nucleic acids to which said primers hybridise.

Two single-stranded sequences will hybridize to each other even if there is not 100% sequence identity between the two sequences, depending on the conditions under which the hybridization reaction occurs and the composition and length of the hybridizing nucleic acid sequences.

Generally, the temperature of hybridization and the ionic strength (such as the Mg²⁺ concentration) of the hybridization buffer will determine the stringency of hybridization. High stringency, such as high hybridization temperature and low salt in hybridization buffers, permits only hybridization between nucleic acid sequences that are highly similar, whereas low stringency, such as lower temperature and high salt, allows hybridization when the sequences are less similar. Calculations regarding hybridization conditions for attaining certain degrees of stringency can be readily carried out by the skilled person and are discussed in Sambrook et al., (1989) Molecular Cloning, second edition, Cold Spring Harbor Laboratory, Plainview, NY (chapters 9 and 11). The skilled person will be able to optimise hybridization conditions according to the results from sensitivity and specificity tests.

The following is an exemplary set of hybridization conditions for use in the present invention:

**Very High Stringency (detects sequences that share at least 90% identity)**

| | |
|---|---|
| Hybridization: | 5x SSC at 65°C for 16 hours |
| Wash twice: | 2x SSC at room temperature (RT) for 15 minutes each |
| Wash twice: | 0.5x SSC at 65°C for 20 minutes each |

**High Stringency (detects sequences that share at least 80% identity)**

| | |
|---|---|
| Hybridization: | 5x-6x SSC at 65°C-70°C for 16-20 hours |
| Wash twice: | 2x SSC at RT for 5-20 minutes each |
| Wash twice: | 1x SSC at 55°C-70°C for 30 minutes each |

**Low Stringency (detects sequences that share at least 50% identity)**

| | |
|---|---|
| Hybridization: | 6x SSC at RT to 55°C for 16-20 hours |
| Wash at least twice: | 2x-3x SSC at RT to 55°C for 20-30 minutes each |

The primers disclosed herein can hybridise to the nucleic acids encoding the Trav12 or TRAV21 alpha chain variable domains or TRBV6 beta chain variable domains under low stringency, high stringency, and very high stringency conditions.

The primer may bind with high stringency to the sequences encoding the alpha and beta chain variable domains. However, the primers may bind to some other loci which have high homology to TRAV12-2, TRAV21 and/or TRVB6.

The TRBV6 encoding nucleic acid of both methods may be a TRBV6-1, a TRBV6-2, a TRBV6-3, TRBV6-5 or a TRBV6-6 gene product.

The nucleic acids of steps i) and ii) are from a natural repertoire. Non-natural mutations may be introduced to the alpha or beta variable domains, prior to step iii) or after step iii), i.e. the nucleic acid sequences may have non-natural mutations introduced prior to being cloned into vectors. Alternatively, the non-natural mutations may be introduced after the cloning steps of iii) and/or iv).

The amplification of the TRAV12-2 or TRAV21 variable domains may be from a pre-prepared cDNA library, itself derived from donor mRNA, with a forward primer designed to specifically bind to the locus of interest. The reverse primer may be designed to specifically bind to (at least partially) the TCR alpha constant region, such that the resulting PCR product contains the TRAV12-2 or TRAV21 nucleic acid sequence, the joining region and at least part of the constant region. Such primer design ensures that the variety and diversity of the alpha chain variable domain CDR3 region is captured, resulting in a large number of unique TCR alpha chain sequences being represented in the library of the invention. Preferably, the donor is human.

Likewise, the amplification of the TRBV6 variable domain may be from an available cDNA library, with a forward primer designed to specifically bind to the locus of interest. The reverse primer may be designed to specifically bind to the TCR beta constant region, such that the resulting PCR product contains a TRBV6 nucleic acid sequence, the joining region (containing the D and J loci) and at least part of the constant region. Such primer design ensures that the variety and diversity of the beta chain variable domain CDR3 region is captured, resulting in a large number of unique TCR beta chain sequences being represented in the library of the invention.

The mRNA is obtained from at least one donor. By "from at least one donor" it is meant that the polypeptide sequence of the alpha or beta chain variable domain is substantially as it would naturally occur in a T cell of the donor from whom the mRNA is obtained.

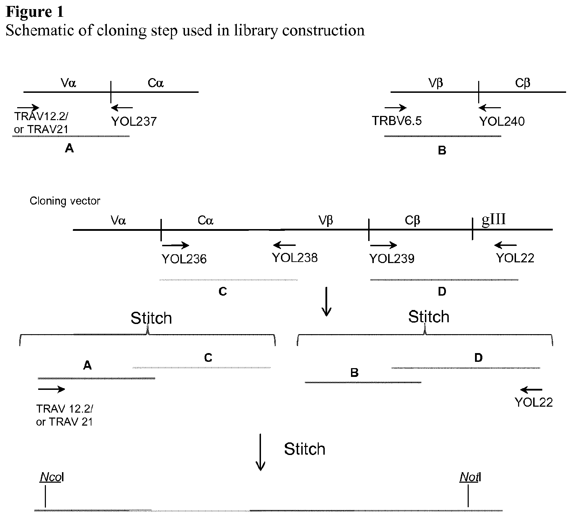

The resulting PCR products may be ligated into a phage vector directly if they contain the complete constant gene sequences, provided that the required ligation or recombination sequences are present in the vector and primer sequences. Alternatively, the alpha and beta PCR products may be stitched together with sequences containing the alpha constant domain gene sequence and the beta constant domain gene sequence respectively, in order to obtain complete TCR chain sequences. The alpha chain and beta chain may be randomly stitched together in order to increase the diversity in the phage library. The complete sequences may then be cloned into a phage vector, to be expressed as one open reading frame (as shown in Figure 1).

Alternatively, other particle display formats may also be used to produce the libraries of the invention. Such methods are known to those of skill in the art and may include, but are not limited, to display on ribosome particles or yeast cells.

These display methods fall into two broad categories, *in-vitro* and *in-vivo* display.

All *in-vivo* display methods rely on a step in which the library, usually encoded in or with the genetic nucleic acid of a replicable particle such as a plasmid or phage replicon is transformed into cells to allow expression of the proteins or polypeptides. (Plückthun (2001) Adv Protein Chem 55 367-403). There are a number of replicon/host systems that have proved suitable for *in-vivo* display of protein or polypeptides. These include the following:
Phage / bacterial cells
plasmid / CHO cells
Vectors based on the yeast 2µm plasmid / yeast cells
bacculovirus / insect cells
plasmid / bacterial cells
retroviral vector/mammalian cells

*In vivo* display methods include cell-surface display methods in which a plasmid is introduced into the host cell encoding a fusion protein consisting of the protein or polypeptide of interest fused to a cell surface protein or polypeptide. The expression of this fusion protein leads to the protein or polypeptide of interest being displayed on the surface of the cell. The cells displaying these proteins or polypeptides of interest can then be subjected to a selection process such as FACS and the plasmids obtained from the selected cell or cells can be isolated and sequenced. Cell surface display systems have been devised for mammalian cells (Higuschi (1997) J Immunol. Methods 202 193-204), yeast cells (Shusta (1999) J Mol Biol 292 949-956) and bacterial cells (Sameulson (2002) J. Biotechnol 96 (2) 129-154). Display of single chain TCRs on the surface of yeast cells is known in the art (WO01/48145)

Numerous reviews of the various in-vivo display techniques have been published. For example, (Hudson (2002) Expert Opin Biol Ther (2001) 1 (5) 845-55) and (Schmitz (2000) 21 (Supp A) S106-S112).

*In-vitro* display methods are based on the use of ribosomes to translate libraries of mRNA into a diverse array of protein or polypeptide variants. The linkage between the proteins or polypeptides formed and the mRNA encoding these molecules is maintained by one of two methods. Conventional ribosome display utilises mRNA sequences that encode a short (typically 40-100 amino acid) linker sequence and the protein or polypeptide to be displayed. The linker sequences allow the displayed protein or polypeptide sufficient space to re-fold without being sterically hindered by the ribosome. The mRNA sequence lacks a 'stop' codon, this ensures that the expressed protein or polypeptide and the RNA remain attached to the ribosome particle. The related mRNA display method is based on the preparation of mRNA sequences encoding the protein or polypeptide of interest and DNA linkers carrying a puromycin moiety. As soon as the ribosome reaches the mRNA/DNA junction translation is stalled and the puromycin forms a covalent linkage to the ribosome. For a review of these two related *in-vitro* display methods see (Amstutz (2001) Curr Opin Biotechnol 12 400-405).

Particularly preferred is the phage display technique which is based on the ability of bacteriophage particles to express a heterologous peptide or polypeptide fused to their surface proteins (Smith (1985) Science 217 1315-1317). The procedure is quite general, and well understood in the art for the display of polypeptide monomers. The display of dimeric proteins such as heterodimeric TCRs is also well established in the art (WO04/044004)

There are two main procedures which apply to both monomeric and dimeric display: Firstly (Method A) by inserting into a vector (phagemid) DNA encoding the heterologous peptide or polypeptide fused to the DNA encoding a bacteriophage coat protein (For example DNA encoding the proteins P3 or P8). The expression of phage particles displaying the heterologous peptide or polypeptide is then carried out by transfecting bacterial cells with the phagemid, and then infecting the transformed cells with a 'helper phage'. The helper phage acts as a source of the phage proteins not encoded by the phagemid required to produce a functional phage particle.

Secondly (Method B), by inserting DNA encoding the heterologous peptide or polypeptide into a complete phage genome fused to the DNA encoding a bacteriophage coat protein. The expression of phage particles displaying the heterologous peptide or polypeptide is then carried out by infecting bacterial cells with the phage genome. This method has the advantage of the first method of being a 'single-step' process. However, the size of the heterologous DNA sequence that can be successfully packaged into the resulting phage particles is reduced. M13, T7 and Lambda are examples of suitable phages for this method.

A variation on (Method B) the involves adding a DNA sequence encoding a nucleotide binding domain to the DNA in the phage genome encoding the heterologous peptide be displayed, and further adding the corresponding nucleotide binding site to the phage genome. This causes the heterologous peptide to become directly attached to the phage genome. This peptide/genome complex is then packaged into a phage particle which displays the heterologous peptide. This method is fully described in WO 99/11785.

The phage particles can then be recovered and used to study the binding characteristics of the heterologous peptide or polypeptide. Once isolated, phagemid or phage DNA can be recovered from the peptide- or polypeptide-displaying phage particle, and this DNA can be replicated via PCR. The PCR product can be used to sequence the heterologous peptide or polypeptide displayed by a given phage particle.

The phage display of single-chain antibodies and fragments thereof, has become a routine means of studying the binding characteristics of these polypeptides. There are numerous books available that review phage display techniques and the biology of the bacteriophage. (See, for example, Phage Display - A Laboratory Manual, Barbas et al., (2001) Cold Spring Harbour Laboratory Press).

A third phage display method (Method C) relies on the fact that heterologous polypeptides having a cysteine residue at a desired location can be expressed in a soluble form by a phagemid or phage genome, and caused to associate with a modified phage surface protein also having a cysteine residue at a surface exposed position, via the formation of a disulphide linkage between the two cysteines. WO 01/ 05950 details the use of this alternative linkage method for the expression of single-chain antibody-derived peptides.

As mentioned above, αβ heterodimeric TCRs of the invention may have an introduced (non-native) disulphide bond between their constant domains. This can be achieved during the method of making the library of the invention by stitching the amplified nucleic acid sequence to a modified constant gene sequence. Such sequences may include those which have a TRAC constant domain sequence and a TRBC1 or TRBC2 constant domain sequence except that Thr 48 of TRAC and Ser 57 of TRBC1 or TRBC2, with reference to IMGT numbering, are replaced by cysteine residues, the said cysteines forming a disulphide bond between the TRAC constant domain sequence and the TRBC1 or TRBC2 constant domain sequence of the TCRs of the library.

With or without the introduced inter-chain bond mentioned in the preceding paragraph, αβ heterodimeric TCRs of the invention may have a TRAC constant domain sequence and a TRBC1 or TRBC2 constant domain sequence, and the TRAC constant domain sequence and the TRBC1 or TRBC2 constant domain sequence of the TCR may be linked by the native disulphide bond between Cys4 of exon 2 of TRAC and Cys2 of exon 2 of TRBC1 or TRBC2.

Alternatively, the TCR alpha chain variable domain and the TCR beta chain variable domain may be expressed as a single chain polypeptide. Such a configuration may include a non-native disulphide bond between mutated amino acid residues.

The invention also provides a method of obtaining a T cell receptor that specifically binds a peptide antigen, comprising screening the library according to the first or second aspects of the invention with the peptide antigen.

The screening may include one or more steps as set out below
a) panning the library using as a target the peptide antigen
b) repeating step a) one or more times
c) screening the phage clones identified in step a) or b)
d) identifying a TCR that specifically binds the peptide antigen.

In accordance with step (b), step (a) may be repeated once, twice, 3 times, 4 times, 5 times, or 6 times. It may be repeated up to 10 times. Step (a) may be repeated up to 20 times or more.

By panning it is meant that the phage clones are allowed to contact an antigen and the bound phage clones separated from the non-bound phage clones. This may include immobilising the antigen on a solid support such as tubes, magnetic beads, column matrices, or BiaCore sensorchips. Antigen attachment may be mediated by non-specific adsorption, or by using a specific attachment tag such as a biotinylated antigen and a streptavidin coated surface. An alternative method may include panning on intact cells. (Hoogenboom, H. R., et al (1998) Immunotechnology, 4(1), 1-20.). The phage clones that do not bind (i.e. phage that do not display a TCR that binds to the antigen) are washed away. The bound phage clones may then be eluted by; enzymatic cleavage of a protease site, such as trypsin, between the TCR beta chain and gene III; extremes of pH; or competition with excess antigen. These phage clones may be taken through further rounds of panning, or on to screening experiments to identify clones with optimal binding characteristics.

The screening may be carried out, for example, by ELISA-based methods with either coated antigen or intact cells and may be in 96-well format; where whole cells are used, screening may be carried out using flow cytometry. Screening for binding affinity and kinetics may be carried out using surface plasmon resonance for example on a BiaCore instrument, or using a quartz crystal microbalance. Screening methods are described in Pande, J., et al. (2010). Biotechnol Adv 28(6): 849-58. As known to those skilled in the art further suitable methods for screening biomolecular interactions of this type are available including: the Octet system from ForteBIO, which utilizes BioLayer Interferometry (BLI) to measure biomolecular interactions in real time and provide information on affinity and kinetics; the Amplified Luminescent Proximity Homogenous Assay (e.g. AlphaScreen™) in which potentially interacting molecules are attached to 'donor' and 'acceptor' beads that have particular fluorescent properties when in close proximity; the Scintillation Proximity Assay in which interactions are assessed by transfer of beta particles between molecules in close proximity; other optical interfacial assays as described in, for example, WO 2004/044004.

Specificity may be determined by testing the identified TCRs for binding to other peptides other than the peptide antigen used to screen the library. If binding occurs to other peptides, the TCR may be considered to be non-specific. Specificity may be assessed using the methods identified above.

The peptide antigen may be a known antigen, such as those described in Bridgeman, J. S., et al. (2012) Immunology, 135(1), 9-18. The method of screening the library of the invention may also be used with novel peptide antigens, in order to identify specifically binding TCRs that may prove useful in therapeutic areas.

Disclosed herein is an isolated cell displaying on its surface a TCR according to the invention, i.e. an isolated T cell receptor (TCR) comprising a TCR alpha chain variable domain comprising a TRAV12-2 gene product or a TRAV21 gene product and a TCR beta chain variable domain comprising a TRBV6 gene product obtained from a library of the first or second aspect of the invention, wherein the TCR specifically binds a peptide antigen. The cell may be a T cell. The cell may be a human, murine or other animal cell.

There are a number of methods suitable for the transfection of T cells with DNA or RNA encoding the TCRs of the invention. (See for example Robbins et al., (2008) J. Immunol. 180: 6116-6131). T cells expressing the TCRs of the invention will be suitable for use in adoptive therapy-based treatment of diseases such as cancers, viral infections, autoimmune diseases, parasitic infections and bacterial infections. As will be known to those skilled in the art there are a number of suitable methods by which adoptive therapy can be carried out. (See for example Rosenberg et al., (2008) Nat Rev Cancer 8 (4): 299-308).

For use in adoptive therapy, the invention also includes cells harbouring a TCR expression vector which comprises nucleic acid encoding the TCR of the invention in a single open reading frame or two distinct open reading frames. Also described are cells harbouring a first expression vector which comprises nucleic acid encoding the alpha chain of a TCR of the invention, and a second expression vector which comprises nucleic acid encoding the beta chain of a TCR of the invention. Alternatively, one vector may express both an alpha and a beta chain of a TCR of the invention.

The TCRs of the invention intended for use in adoptive therapy may be glycosylated when expressed by the transfected T cells. As is well known, the glycosylation pattern of transfected TCRs may be modified by mutations of the transfected gene (Kuball J et al. (2009), J Exp Med 206(2):463-475).

For administration to patients, T cells transfected with TCRs of the invention may be provided in pharmaceutical composition together with a pharmaceutically acceptable carrier. Cells in accordance with the invention will usually be supplied as part of a sterile, pharmaceutical composition which will normally include a pharmaceutically acceptable carrier. This pharmaceutical composition may be in any suitable form, (depending upon the desired method of administering it to a patient). It may be provided in unit dosage form, will generally be provided in a sealed container and may be provided as part of a kit. Such a kit would normally (although not necessarily) include instructions for use. It may include a plurality of said unit dosage forms. Suitable compositions and methods of administration are known to those skilled in the art, for example see, Johnson et al. Blood (114):535-46 (2009*),* with reference to clinical trial numbers NCI-07-C-0175 and NCI-07-C-0174.

The pharmaceutical composition may be adapted for administration by any appropriate route such as a parenteral (including subcutaneous, intramuscular, intravenous, or intraperitoneal), inhalation or oral route. Such compositions may be prepared by any method known in the art of pharmacy, for example by mixing the active ingredient with the carrier(s) or excipient(s) under sterile conditions.

Dosages of the substances of the present invention can vary between wide limits, depending upon the disease or disorder to be treated such as cancer, viral infection, autoimmune disease, bacterial infection or parasitic infection, the age and condition of the individual to be treated, etc. For example, a suitable dose range for an ImmTAC reagent (a soluble TCR fused to an anti-CD3 domain) may be between 25 ng/kg and 50 µg/kg. A physician will ultimately determine appropriate dosages to be used.

TCRs of the inventions may also be may be labelled with an imaging compound, for example a label that is suitable for diagnostic purposes. Such labelled high affinity TCRs are useful in a method for detecting a TCR ligand selected from CD1-antigen complexes, bacterial superantigens, and MHC-peptide/superantigen complexes which method comprises contacting the TCR ligand with a high affinity TCR (or a multimeric high affinity TCR complex) which is specific for the TCR ligand; and detecting binding to the TCR ligand. In tetrameric high affinity TCR complexes (formed, for example) using biotinylated heterodimers) fluorescent streptavidin (commercially available) can be used to provide a detectable label. A fluorescently-labelled tetramer is suitable for use in FACS analysis, for example to detect antigen presenting cells carrying the peptide for which the high affinity TCR is specific.

A high affinity TCR (or multivalent complex thereof) of the present invention may alternatively or additionally be associated with (e.g. covalently or otherwise linked to) a therapeutic agent which may be, for example, a toxic moiety for use in cell killing, or an immunostimulating agent such as an interleukin or a cytokine. A multivalent high affinity TCR complex of the present invention may have enhanced binding capability for a TCR ligand compared to a non-multimeric wild-type or high affinity T cell receptor heterodimer. Thus, the multivalent high affinity TCR complexes according to the invention are particularly useful for tracking or targeting cells presenting particular antigens *in vitro* or *in vivo,* and are also useful as intermediates for the production of further multivalent high affinity TCR complexes having such uses. The high affinity TCR or multivalent high affinity TCR complex may therefore be provided in a pharmaceutically acceptable formulation for use *in vivo.*

High affinity TCRs of the invention may be used in the production of soluble bi-specific reagents. These may be ImmTAC reagents. ImmTAC reagents comprise a soluble TCR, fused via a linker to an anti-CD3 specific antibody fragment. Further details including how to produce such reagents are described in WO10/133828.

Preferred or optional features of each aspect of the invention are as for each of the other aspects *mutatis mutandis.* Accordingly, although the present invention and its advantages have been described in detail, it should be understood that various changes, substitutions and alterations can be made herein without departing from the scope of the invention as defined in the appended claims.

The present invention will be further illustrated in the following Examples which are given for illustration purposes only and are not intended to limit the invention in any way.

### Examples

### Example 1

### Preparation of cDNA for construction of TRAV12.2/TRBV6* and TRAV21/TRBV6* native TCR phage display libraries

### Isolation of mRNA from peripheral blood lymphocytes (PBLs)

RNA was extracted from a pool of approximately 30 million PBLs obtained from three donors of known HLA type. RNA extraction was carried out using TRI reagent (Sigma, Cat. No. T9424), in accordance with the manufacturer's recommended protocol. mRNA was subsequently isolated using µMACS™ mRNA Isolation Kits (Miltenyi, Cat. No. 130-075-101), as directed by the manufacturer.

### Preparation of cDNA from mRNA

cDNA was synthesised from the mRNA using SMARTScribe™ Reverse Transcriptase (Clontech, 639536), in accordance with the manufacturer's recommended protocol. cDNA was further purified using S.N.A.P. Gel Purification Kit (Invitrogen, 45-0078).

### Example 2

### Phage library construction

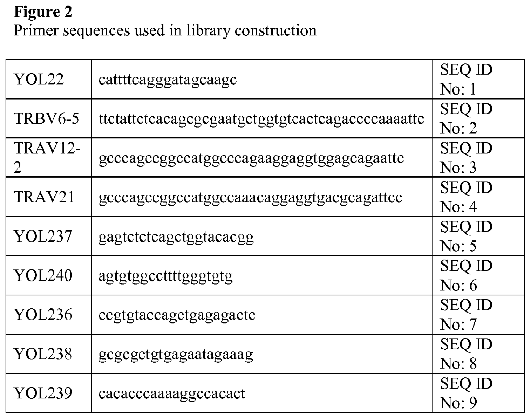

An outline of the library construction is shown in Figure 1 and the corresponding primer sequences detailed in Figure 2. TCR chains were amplified by PCR from purified cDNA using TRAV12.2, TRAV21 or TRBV6* forward primers and reverse primers which anneal within either the TRAC (primer YOL237) or the TRBC regions (primer YOL 240). The primer sets were designed with reference to the known sequences of human TCR chains (T Cell Receptor Facts Book, Lefranc and Lefranc, Publ. Academic Press 2001). The resulting PCR products comprised the full variable domain sequence and a truncated constant domain (labelled A and B in Figure 1). The remaining C-terminal section of the TRAC and TRBC2 domains, containing the non-native cysteine residues, were amplified by PCR from a separate cloning vector using the primers YOL236 and YOL238 for TRAC, and YOL239 and YOL22 for TRBC2 (labelled C and D in Figure 1). Purified A/C and B/D fragments were then stitched together in separate reactions via their overlapping primer regions (YOL237/YOL236 and YOL240/YOL239 respectively). The resulting A-C and B-D fragments were gel purified and stitched together via overlap PCR using TRAV12.2/ 21 forward primer and YOL22 reverse primer, with the TRBV6* and YOL238 primer regions providing the overlapping sequence. This final stitching reaction results in random recombination between alpha chains and beta chains. The Nco1/Not1 restriction sites were used to insert the randomly recombined chains into a suitable phagemid vector, termed pIM672 (pIM672 is based on the pEX922 vector previously described (see WO2005116074)), which was then used to transform highly transformation efficient electro-competent TG1 *E. coli* cells. Cultures were plated on 2xTYag (EzMix, Sigma, Cat. No. Y2627 plus 100 µg/ml ampicillin and 2% glucose) agar plates overnight at 30°C, and the resultant cell lawns scraped into a small volume of 2xTYag medium containing 20% glycerol and 2% glucose. Glycerol stocks of the libraries were stored at -80°C.

### Example 3

### Library propagation and panning

### Propagation of phage particles

An aliquot of phage library glycerol stock, sufficient to cover the diversity of the library, (TRAV12.2/TRBV6 and TRAV21/TRBV6) was used to inoculate 2xYTag media, to an initial OD600 of 0.05. The cultures were then incubated to an OD600 of about 0.5. Helper phage were then added at an infection ratio of ∼ 20:1 phage to *E. coli,* The cultures were then mixed by inverting and incubated for 30 min at 37°C. The cultures were centrifuged and the pellets resuspended in 2xYTak (as 2xYTag but in the absence of glucose and with the addition of 50 µg/ml kanamycin) and subsequently incubated at 26°C for 16 h with shaking.

### Isolation of phage particles

The cultures were pooled, centrifuged and the supernatant collected and filtered at 0.45 µm. The eluate was mixed with 7ml PEG/NaCl (20%PEG-8000 (Sigma Cat. No. 5413), 2.5M NaCl) and incubated on ice for 30 min. The sample was then pelleted and the supernatant discarded. The pellet was resuspended in 10 ml in PBS (Dulbeccos Sigma Cat. No. D8537 - no Mg, no Ca) and re- centrifuged. The resulting supernatant was collected, mixed with 5ml PEG/NaCl and stored on ice for 30 min. After centrifuging, the pellet was resuspended in 3 ml PBS, re-centrifuged, and the supernatant collected. An estimate of the phage concentration was determined using a Nanodrop spectrophotometer, where the number of phage per ml = OD260 x (22.14 x10¹⁰). A library prepared according to this method was calculated to contain 6.8 x 10¹²/ml phage particles.

### Panning

Purified phage particles were mixed with 3% MPBS buffer (PBS (Dulbeccos Sigma Cat. No. D8537 - no Mg, no Ca) plus 3% milk powder, previously incubated with streptavidin-coated paramagnetic beads, and then treated with 15mM EDTA followed by extensive dialysis, and finally filtered at 0.22 µm) and incubated at room temperature for 1 h. For pan 2 and pan 3 after the first 30 minutes an irrelevant non-biotinylated peptide-HLA construct was added for negative selection at a final concentration of 2µM and incubation continued for a further 30 min. 10% (v/v) Tween-20 was then added plus 100nM or 1µM biotinylated peptide-HLA. Samples were mixed at room temperature for 60 min. Phage-biotinylated-HLA complexes were rescued by the addition of streptavidin-coated paramagnetic beads pre-blocked in 3% MPBS buffer, and incubated at room temperature for 7 min. After capture, beads were isolated using a magnetic concentrator (Dynal) and washed three times with 3% MPBS (not EDTA treated) and twice with PBS-0.1%Tween. Phage particles were eluted in 0.5ml TBSC (10 mM Tris, pH7.4, 137 mM NaCl, 1 mM CaCl₂ and 0.1 mg/ml trypsin) for 25 min at room temperature and 5 min at 37°C with gentle rotation.

Eluted phage particles were used to infect early log phase TG1 *E. coli* cells. Cultures were incubated for 37°C for 30 min and subsequently plated out onto YTEag (lOg Tryptone, 5g yeast extract, 8g NaCl, 15g Bacto-Agar in 1L MQ-water, plus 100µg/ml ampicillin, and 2% glucose) in serial dilutions of 1 µl, 0.1 µl and 0.01 µl. The remaining culture was concentrated and also plated onto YTEag. Plates were incubated at 30°C for 16 h. The following day colonies from the plates were added to 2xTYag, frozen on dry ice and stored at -80°C for the next round of panning. Colonies from each selection were analysed by PCR to check for full-length inserts.

After the third round of selection, colonies were scrapped from agar plates and used to inoculate sterile 2xTYag in a 96 well Cellstar cell culture plate at one clone per well. Plates were incubated at 26°C for 16 h with shaking. These cultures were then used to inoculate fresh 2xTYag media in 96 well plates and incubated for 30 min at 37°C with shaking until OD600 = 0.5. Helper phage were then added to each well at 20:1 phage - *E .coli* infection ratio and the plates incubated for 30 min at 37°C without shaking. Pellets were collected by centrifugation and resuspended in 2xYTak. Plates were incubated for 16 h at 26°C with shaking. Cells were then pelleted and supernatant collected for ELISA screening.

### Example 4

### Detection of phage bearing antigen-binding TCR by ELISA screening

### Method

Phage clones bound to peptide-HLA complex were identified by ELISA screening. ELISA plates were prepared using biotinylated peptide-HLA(s) of interest and a control peptide-HLA. Phage particles were added in duplicate to the ELISA plate such that one sample was added to a well containing the peptide-HLA of interest and the other was added to an adjacent control well. Detection was carried out using an anti-Fd antibody (Sigma, Cat. No. B7786) followed by a monoclonal anti-rabbit IgG peroxidase conjugate (gamma chain specific clone RG96) (Sigma, Cat. No. A1949). Bound antibody was detected using the KPL labs TMB Microwell peroxidase Substrate System (Cat. No. 50-76-00). The appearance of a blue colour in the wells indicated the phage clone had bound to the HLA antigen. A lack of colour in the corresponding control wells indicated that the binding was specific.

### Results

A TRAV12.2/21 TRBV6* library, was prepared using the methods described in Examples 1, 2, and 3, except that the two library cultures [TRAV12.2 TRBV6* and TRAV21 TRBV6*] were pooled prior to phage particle isolation and panning steps described in Example 3. Of the three donors used to prepare the library one was HLA-A2 positive, HLA-24 negative, one was HLA-24 positive, HLA-A2 negative and the third was negative for both HLA-A2 and HLA-A24). ELISA screening was carried out as described above. Positive ELISA results were obtained for 12 different HLA-A2 peptides and 1 HLA-A24 peptide, over 2 panning campaigns comprising a total of 16 different peptide HLA complexes. These data demonstrate that the library of the invention can be used to isolate antigen binding TCRs.

A second TRAV12.2/21 TRBV6* library was prepared using the same method as described in Examples 1, 2, and 3, except that the two library cultures [TRAV12.2 TRBV6* and TRAV21 TRBV6*] were pooled prior to phage particle isolation and panning steps described in Example 3, and in addition, all three donors used to prepare the library were HLA-A2 HLA-A24 positive. ELISA screening was carried out using the method described above. Positive ELISA results were obtained for 16 different HLA-A2 peptides and 1 HLA-A24 peptide, over one panning campaign which included 18 different peptide HLA complexes. These data demonstrate that the library of the invention can be used to isolate antigen binding TCRs.

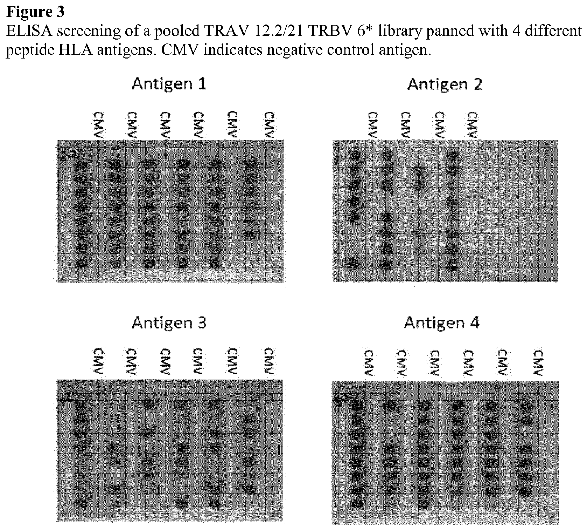

Figure 3 shows the results from ELISA screening after panning with four different HLA-A2 peptides.

### Example 5

### ELISA screening from panning a TRAV12.2/21 TRBV6* library prepared from a HLA-A2/HLA-A24 negative donor

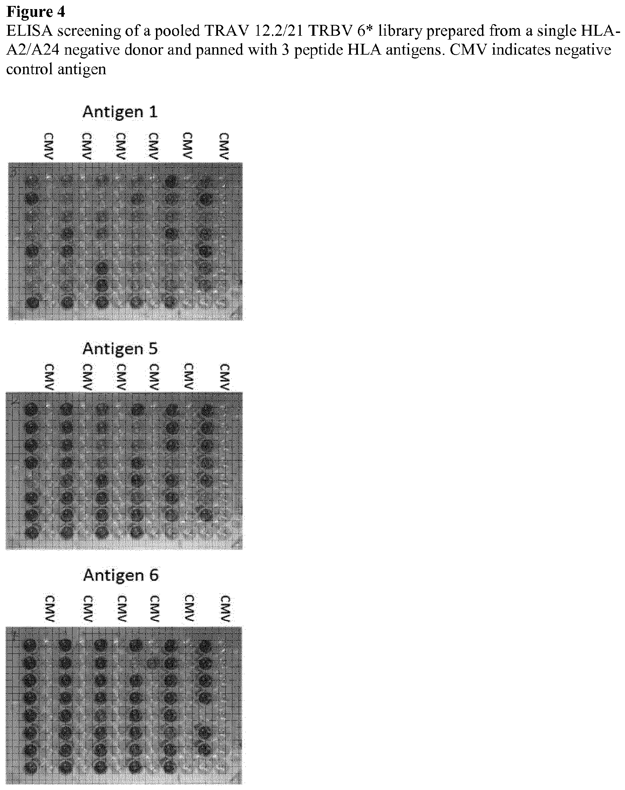

To confirm that antigen binding TCRs could be isolated from a library prepared from a HLA-A2/HLA-A24 negative donor, a third TRAV12-2/21/TRBV6* library was created using the same method as described in Examples 1, 2, and 3 except that the two library cultures [TRAV12.2 TRBV6* and TRAV21 TRBV6*] were pooled prior to phage particle isolation and panning steps described in Example 3, and in addition cDNA was produced from a single HLA-A2 and HLA-A24 negative donor. ELISA screening was carried out using the method of Example 4. From a single panning campaign (which included three rounds of panning) including eight antigens, ELISA results were obtained for four different HLA-A2 peptides and four different HLA-A24 peptides. Figure 4 show results from ELISA screening after panning with three different antigens.

### Example 6

### ELISA screening from panning individual TRAV12.2 TRBV6* and TRAV21 TRBV6* libraries

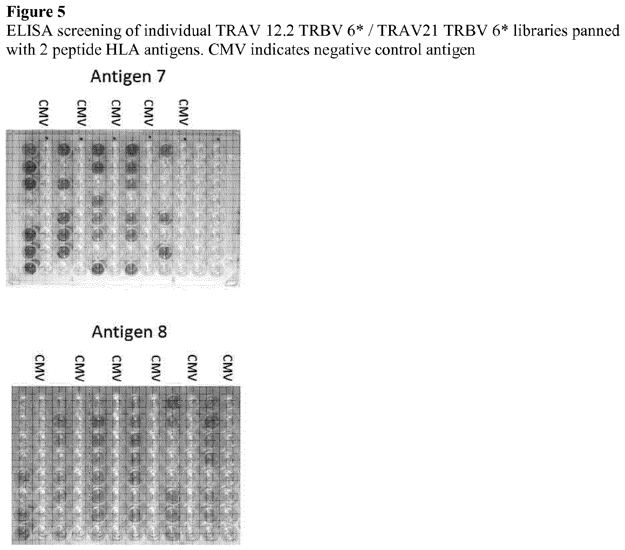

Individual TRAV12.2 TRBV6* and TRAV21 TRBV6* libraries were prepared using the methods described in Example 1, 2, and 3. The libraries were not pooled prior to phage isolation and were panned individually. Positive ELISA results were obtained from both TRAV12.2 TRBV6* and TRAV21 TRBV6* libraries. Figure 5 show results from ELISA screening after panning with two different antigens.

### Example 7

### ELISA screening from panning individual TRAV12.2 TRBV6* and TRAV21 TRBV6* created from a commercial mRNA source

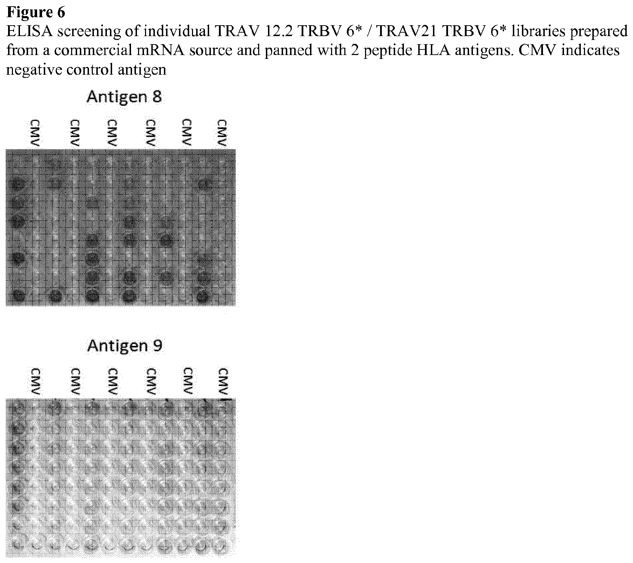

A combined TRAV12-2/21/TRBV6* library was created using the same method as described in Examples 1, 2, and 3 except cDNA was prepared from an mRNA pool obtained from a commercial source (Clontech Cat. No. 636170; Lot No: 1304103A. Donors were 380 males (ages 18-40) and 170 females (ages 18-40). All donors were tested for HIV-I,II, Hepatitis B and syphilis). 50ug mRNA was used to produce cDNA. The libraries were not pooled prior to phage isolation and were panned individually. ELISA screening was carried out using the method of Example 4. Positive ELISA results were obtained from both TRAV12.2 TRBV6* and TRAV21 TRBV6* libraries. Figure 6 show results from ELISA screening after panning with one antigen.

### Example 8

### Analysis of TCR sequences

The DNA sequence of the TCRs from ELISA positive phage clones can be obtained by sequencing using methods known to those skilled in the art. In some cases the TCRs converge to a single sequence, in other cases more than one TCR sequence is identified. For example, from the ELISA plates shown in Figure 3 three different TCR sequences were obtained for Antigen 1, and four different TCR sequences were obtained for Antigen 2. These results demonstrate that multiple TCRs specific for HLA restricted antigens can be isolated from the library of the invention in a single panning campaign.

### Example 9

### Specificity of library TCRs

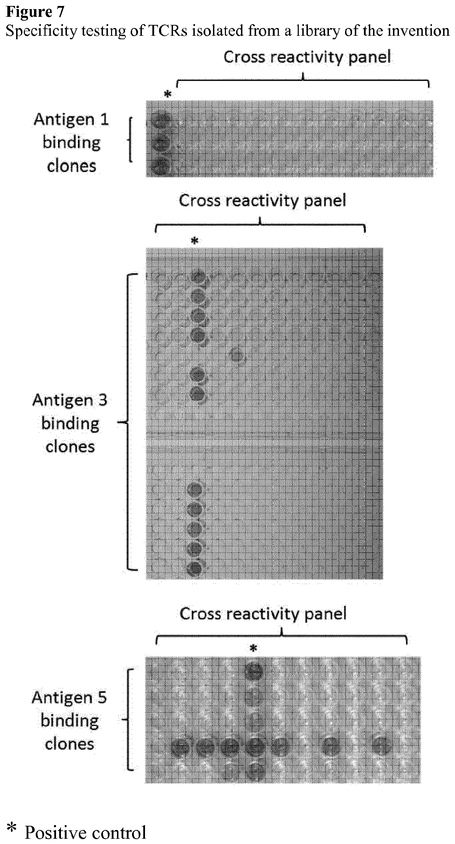

TCRs can be further tested to determine their specificity for the peptide-HLA complex they were selected for during panning. This may be achieved using the same ELISA approach as described above, and a panel of different peptide-HLA complexes. Figure 7 shows results from further specificity tests with TCRs, isolated from ELISA screening, which were selected for binding to Antigen 1, Antigen 3 or Antigen 5. This demonstrates that TCRs with high specificity for antigen can be isolated from the library.

### Example 10

### Biacore analysis of TCRs obtained from the library

### Method

The affinity for antigen of the TCRs isolated from the library was determined by surface plasmon resonance using a BIAcore 3000 instrument and reported in terms of an equilibrium dissociation constant (KD). The TCRs sequences obtained from the phage clones were used to produce soluble versions of the TCRs using the method described in Boulter, et al., Protein Eng, 2003. 16: 707-711. Biotinylated specific and control pMHC monomers were prepared as described in Garboczi, et al. Proc Natl Acad Sci U S A 1992. 89: 3429-3433 and O'Callaghan, et al., Anal Biochem 1999. 266: 9-15, and immobilized on to a streptavidin-coupled CM-5 sensor chips. All measurements were performed at 25°C in PBS buffer (Sigma) supplemented with 0.005% Tween (Sigma) at a constant flow rate. To measure affinity, serial dilutions of the soluble TCRs were flowed over the immobilized pMHCs and the response values at equilibrium were determined for each concentration. Equilibrium dissociation constants (KD) were determined by plotting the specific equilibrium binding against protein concentration followed by a least squares fit to the Langmuir binding equation, assuming a 1:1 interaction.

### Results

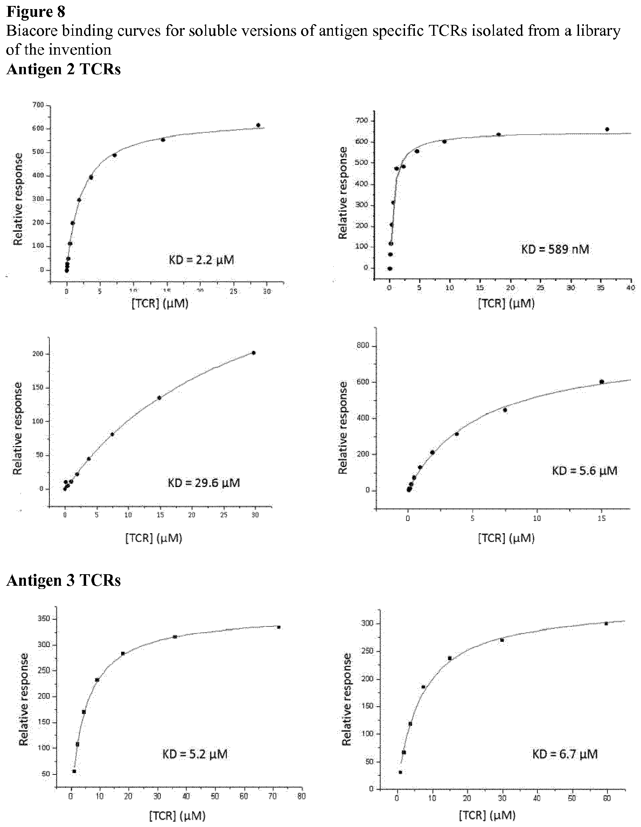

Figure 8 shows the binding curves and equilibrium dissociation constant (KD) for the four TCRs obtained for Antigen 2 and two of the TCRs obtained for Antigen 3. This demonstrates TCRs isolated from the library have a useful affinity for the corresponding antigen.

### Example 11

### TCRs obtained from the library can be affinity enhanced

A specific peptide-HLA TCR isolated from a TRAV 12.2/21 TRBV6* library had an affinity (K_{D}) of 25 µM as determined by the surface plasmon resonance method described in Example 10. To obtain higher affinity variants of this TCR the variable domain sequence of the alpha and beta chains were mutated. Methods for producing mutated high affinity TCR variants such as phage display and site directed mutagenesis and are known to those in the art (for example see WO04044004 and Li et al, (2005) Nature Biotech 23 (3): 349-354). The affinity and half-life of the TCR variants for antigen were analysed by single-cycle kinetics using BIAcore3000. Specific and control pHLAs were immobilized to different flow cells and increasing concentrations of TCRs were injected. Global fit was performed using the BIAevaluation software to simultaneously derive kₒₙ, k_{off}, K_{D} and half-life values.

### Results

A number of variants with mutations in either the alpha (A) or beta (B) chain and having higher affinity for antigen were isolated

| TCR variant | KD (nM) | Half life |
|---|---|---|
| A1 | 14 | 6.8 min |
| A2 | 33 | 6 min |
| A3 | 80 | 47 s |
| A5 | 33 | 34.5 min |
| A8 | 340 | 2 s |
| A9 | 81 | 36 s |
| A10 | 7 | 9.4 min |
| A14 | 13 | 2.9 min |
| B1 | 85 | 27 s |
| B2 | 87 | 26 s |
| B3 | 22 | 63 s |
| B4 | 73 | 21 s |
| B5 | 18 | 73 s |
| B8 | 220 | 43 s |
| B9 | 860 | 1.8 s |
| B10 | 1100 | 1.6 s |

Several beta chain variants were fused to an anti-CD3 antibody and refolded with selected alpha chain variants to produce soluble TCRs fusion proteins, suitable for use in immunotherapeutic applications. Further details describing the production of such TCR fusion proteins are found in WO10133828. Affinity and half-life measurements were performed as described above.

| TCR Fusion | KD (pM) | Half life (h) |
|---|---|---|
| A2B1 | 24 | 20 |
| A2B5 | 20 | 24 |
| A2B3 | 39 | 15 |
| A10B1 | 18 | 20 |
| A10B3 | 25 | 13 |
| A10B5 | 19 | 16 |

### Comparative Example

### Isolating antigen binding TCRs from fresh blood

Prior to the construction of the library of the invention, antigen-specific TCRs were obtained from T cells isolated from fresh donor blood, after stimulation with a peptide-HLA antigen of interest. Experiments were divided into cloning campaigns, involving up to 20 different antigens and fresh blood obtained from between 12 and 20 individual donors. TCR chains were amplified from the responding T cells and used to produce soluble TCRs. Antigen binding was demonstrated by the Biacore method of Example 10.

### Method

T cells, B cells and dendritic cells were obtained from 200 ml fresh donor blood. Three rounds of stimulations were performed, first with autologous DCs and then with autologous B cells pulsed with the panel of antigenic peptides of interest. Activated T cells were detected via an ELISpot assay (BD Biosciences) and T2 cells pulsed with either the antigenic peptides of interest, or an irrelevant control antigen. Responding cells were stained for interferon gamma (IFNγ) and sorted by IFNγ or CD8 expression. Antigen-binding cell lines were confirmed by ELISpot assay and tetramer staining. TCR chains from validated clones were amplified by rapid amplification of cDNA ends (RACE) and cloned into an *E. coli* expression vector. TCRs were purified and refolded from inclusion bodies. Antigen binding was confirmed by binding on Biacore.

### Results

From five cloning campaigns carried out over several years, fewer than 10 specific antigen-binding TCRs were identified. Therefore, the library of the invention is much more efficient for obtaining TCRs than the approach used in this example.

With reference to the antigens exemplified above, Antigens 1 and 2 were included in two campaigns (4 + 5, and 3 + 4, respectively) and Antigens 3 and 4 were included in one campaign (4 and 5, respectively). No TCRs specific for Antigens 1, 2 and 3 were obtained using this method. A single TCR that bound Antigen 4 has been obtained. This demonstrates that even for the same antigens, the library of the invention is much more efficient for obtaining TCRs than the approach used in this example.

The three HLA-A2/ HLA-A24 blood donors, used to create the second library of Example 4, described above, have been used in some of the cloning campaigns. One was used campaign 1, 2, 4 and 5; the second was used in campaign 4 and 5; and a third was used in campaign 5. This demonstrates that even using the same donors, the library of the invention is much more efficient for obtaining TCRs than the approach used in this example.

As demonstrated, many cloning campaigns have been carried out in order to try to isolate specific antigen-binding TCRs, with very limited success. The campaigns were labour intensive, unreliable and involved the collection of multiple blood donations from many donors. The results presented herein show that the present invention allows the quick, effective and reliable identification of multiple antigen-binding TCRs, which were unable to be identified or very difficult to identify with previously known techniques.

Having thus described in detail preferred embodiments of the present invention, it is to be understood that the invention defined by the above paragraphs is not to be limited to particular details set forth in the above description as many apparent variations thereof are possible without departing from the scope of the present invention.

## Claims

1. A library of particles, the library displaying a plurality of different T cell receptors (TCRs), wherein the plurality of TCRs consists essentially of TCRs comprising an alpha chain comprising an alpha chain variable domain from a natural repertoire and a beta chain comprising a beta chain variable domain from a natural repertoire, wherein the alpha chain variable domain comprises a TRAV12-2 or a TRAV21 gene product and the beta chain variable domain comprises a TRBV6 gene product.

2. A library of particles, the library displaying a plurality of different T cell receptors (TCRs), wherein the plurality of TCRs consists essentially of TCRs comprising an alpha chain comprising an alpha chain variable domain from a natural repertoire and a beta chain comprising a beta chain variable domain from a natural repertoire, wherein the alpha chain variable domain comprises a TRAV12-2 or a TRAV21 gene product and the beta chain variable domain comprises a TRBV6 gene product and wherein at least a portion of the TCRs comprise an alpha chain variable domain and/or a beta chain variable domain comprising a non-natural mutation.

3. The library according to claim 1 or claim 2, wherein the TRBV6 gene product is a TRBV6-1, a TRBV6-2, a TRBV6-3, a TRBV6-5 or a TRBV6-6 gene product.

4. The library according to any one of claims 1 to 3 wherein the alpha chain variable domain and the beta chain variable domain are displayed as a single polypeptide chain.

5. The library according to any one of claims 1 to 4 wherein the TCRs comprise a non-native disulphide bond or a native disulphide bond between a constant region of the alpha chain and a constant region of the beta chain.

6. The library according to any one of claims 1 to 5 wherein the particles are phage particles, ribosomes, yeast cells or mammalian cells.

7. A non-natural isolated T cell receptor (TCR) comprising a TCR alpha chain variable domain comprising a TRAV12-2 gene product or a TRAV21 gene product and a TCR beta chain variable domain comprising a TRBV6 gene product obtained from a library according to claim 2.

8. Use of a library according to any one of claims 1 to 6 to identify a TCR that specifically binds to a peptide antigen.

9. A method of obtaining a T cell receptor that specifically binds a peptide antigen, comprising screening the library according claim 1 or claim 2 with the peptide antigen, the method comprising:
a) panning the library using as a target the peptide antigen;
b) repeating step a) one or more times;
c) screening the phage clones identified in step a) or b); and
d) identifying a TCR that specifically binds the peptide antigen.

10. A nucleic acid encoding a TCR alpha chain variable domain and a beta chain variable domain of the TCR according to claim 7.

11. A method of making a library of particles, the library displaying a plurality of different TCRs, the method comprising:
i) obtaining a plurality of nucleic acids from a natural repertoire that encode different TRAV12-2 or TRAV21 alpha chain variable domains;
ii) obtaining a plurality of nucleic acids from a natural repertoire that encode different TRBV6 beta chain variable domains;
iii) cloning the TRAV12-2 or TRAV21 alpha chain variable domain encoding nucleic acids into expression vectors;
iv) cloning the TRBV6 beta chain variable domain encoding nucleic acids into the same or different vectors; and
v) expressing the vectors in particles, thereby generating a library consisting essentially of TCRs comprising an alpha chain variable domain and a beta chain variable domain encoded by the nucleic acids.

12. A method of making a library of particles, the library displaying a plurality of different TCRs, the method comprising:
i) obtaining a plurality of nucleic acids from a natural repertoire that encode different TRAV12-2 or TRAV21 alpha chain variable domains using primers that hybridise to nucleic acids encoding TRA12-2 or TRAV21 alpha chain variable domains;
ii) obtaining a plurality of nucleic acids from a natural repertoire that encode different TRBV6 beta chain variable domains using primers that hybridise to nucleic acids encoding TRAV6 beta chain variable domains;
iii) cloning the TRAV12-2 or TRAV21 alpha chain variable domain encoding nucleic acids into expression vectors;
iv) cloning the TRBV6 beta chain variable domain encoding nucleic acids into the same or different vectors; and
v) expressing the vectors in particles, thereby generating a library consisting essentially of TCRs comprising an alpha chain variable domain and a beta chain variable domain encoded by the nucleic acids to which said primers hybridise.

13. The method of any one of claims 11 or 12, comprising a further step of introducing non-natural mutations to the nucleic acids, optionally wherein non-natural mutations are introduced to the nucleic acids prior to step iii).

14. A method according to any one of claims 11 to 13, wherein the TCR alpha chain variable domain and the TCR beta chain variable domain are expressed as a single chain polypeptide.

15. A method of obtaining a T cell receptor that specifically binds a peptide antigen, comprising screening the library according to any one of claims 1 to 6 with the peptide antigen.

16. A particle displaying on its surface a TCR according claim 7, optionally wherein the particle is a phage particle, a ribosome, a yeast cell or a mammalian cell.

## Patentansprüche

1. Bibliothek von Partikeln, wobei die Bibliothek mehrere unterschiedliche T-Zell-Rezeptoren (TCRs) präsentiert, wobei die mehreren TCRs im Wesentlichen aus TCRs bestehen, die eine Alpha-Kette umfassend eine Alpha-Kette-variable-Domäne aus einem natürlichen Repertoire und eine Beta-Kette umfassend eine Beta-Kette-variable-Domäne aus einem natürlichen Repertoire umfassen, wobei die Alpha-Kette-variable-Domäne ein TRAV12-2- oder ein TRAV21-Gen-produkt umfasst und die Beta-Kette-variable-Domäne ein TRBV6-Genprodukt umfasst.

2. Bibliothek von Partikeln, wobei die Bibliothek mehrere unterschiedliche T-Zell-Rezeptoren (TCRs) präsentiert, wobei die mehreren TCRs im Wesentlichen aus TCRs bestehen, die eine Alpha-Kette umfassend eine Alpha-Kette-variable-Domäne aus einem natürlichen Repertoire und eine Beta-Kette umfassend eine Beta-Kette-variable-Domäne aus einem natürlichen Repertoire umfassen, wobei die Alpha-Kette-variable-Domäne ein TRAV12-2- oder ein TRAV21-Gen-produkt umfasst und die Beta-Kette-variable-Domäne ein TRBV6-Genprodukt umfasst und wobei die TCRs wenigstens zum Teil eine Alpha-Kette-variable-Domäne und/oder eine Beta-Kette-variable-Domäne umfassen, die eine nicht natürliche Mutation umfasst.

3. Bibliothek nach Anspruch 1 oder Anspruch 2, wobei es sich bei dem TRBV6-Genprodukt um ein TRBV6-1-, ein TRBV6-2-, ein TRBV6-3-, ein TRBV6-5- oder ein TRBV6-6-Genprodukt handelt.

4. Bibliothek nach einem der Ansprüche 1 bis 3, wobei die Alpha-Kette-variable-Domäne und die Beta-Kette-variable-Domäne als eine einzelne Polypeptidkette präsentiert werden.

5. Bibliothek nach einem der Ansprüche 1 bis 4, wobei die TCRs eine nicht native Disulfidbindung oder eine native Disulfidbindung zwischen einer konstanten Region der Alpha-Kette und einer konstanten Region der Beta-Kette umfassen.

6. Bibliothek nach einem der Ansprüche 1 bis 5, wobei es sich bei den Partikeln um Phagenpartikel, Ribosomen, Hefezellen oder Säugerzellen handelt.

7. Nicht natürlicher isolierter T-Zell-Rezeptor (TCR), umfassend eine TCR-Alpha-Kette-variable-Domäne, die ein TRAV12-2-Genprodukt oder ein TRAV21-Genprodukt umfasst, und eine TCR-Beta-Kette-variable-Domäne, die ein TRBV6-Genprodukt umfasst, erhalten aus einer Bibliothek nach Anspruch 2.

8. Verwendung einer Bibliothek nach einem der Ansprüche 1 bis 6 zur Identifizierung eines TCR, der an ein Peptidantigen spezifisch bindet.

9. Verfahren zur Gewinnung eines T-Zell-Rezeptors, der an ein Peptidantigen spezifisch bindet, umfassend Screening der Bibliothek nach Anspruch 1 oder Anspruch 2 mit dem Peptidantigen, wobei das Verfahren Folgendes umfasst:
a) Panning der Bibliothek unter Verwendung des Peptidantigens als Ziel;
b) ein- oder mehrmaliges Wiederholen von Schritt a) ;
c) Screening der in Schritt a) oder b) identifizierten Phagenklone; und
d) Identifizieren eines TCR, der das Peptidantigen spezifisch bindet.

10. Nukleinsäure, codierend eine TCR-Alpha-Kette-variable-Domäne und/oder eine Beta-Kette-variable-Domäne des TCR nach Anspruch 7.

11. Verfahren zum Herstellen einer Bibliothek von Partikeln, wobei die Bibliothek mehrere unterschiedliche TCRs präsentiert, wobei das Verfahren Folgendes umfasst:
i) Gewinnen mehrerer Nukleinsäuren aus einem natürlichen Repertoire, die unterschiedliche TRAV12-2- oder TRAV21-Alpha-Kette-variable-Domänen codieren;
ii) Gewinnen mehrerer Nukleinsäuren aus einem natürlichen Repertoire, die unterschiedliche TRBV6-Beta-Kette-variable-Domänen codieren;
iii) Klonieren der TRAV12-2- oder TRAV21-Alpha-Kette-variable-Domänen codierenden Nukleinsäuren in Expressionsvektoren;
iv) Klonieren der TRBV6-Beta-Kette-variable-Domänen codierenden Nukleinsäuren in die gleichen oder andere Vektoren; und
v) Exprimieren der Vektoren in Partikeln, wodurch eine Bibliothek erzeugt wird, die im Wesentlichen aus TCRs besteht, die eine Alpha-Kette-variable-Domäne und eine Beta-Kette-variable-Domäne codiert durch die Nukleinsäuren umfassen.

12. Verfahren zum Herstellen einer Bibliothek von Partikeln, wobei die Bibliothek mehrere unterschiedliche TCRs präsentiert, wobei das Verfahren Folgendes umfasst:
i) Gewinnen mehrerer Nukleinsäuren aus einem natürlichen Repertoire, die unterschiedliche TRAV12-2- oder TRAV21-Alpha-Kette-variable-Domänen codieren, unter Verwendung von Primern, die an TRA12-2- oder TRAV21-Alpha-Kette-variable-Domänen codierende Nukleinsäuren hybridisieren;
ii) Gewinnen mehrerer Nukleinsäuren aus einem natürlichen Repertoire, die unterschiedliche TRBV6-Beta-Kette-variable-Domänen codieren, unter Verwendung von Primern, die an TRAV6-Beta-Kette-variable-Domänen codierende Nukleinsäuren hybridisieren;
iii) Klonieren der TRAV12-2- oder TRAV21-Alpha-Kette-variable-Domänen codierenden Nukleinsäuren in Expressionsvektoren;
iv) Klonieren der TRBV6-Beta-Kette-variable-Domänen codierenden Nukleinsäuren in die gleichen oder andere Vektoren; und
v) Exprimieren der Vektoren in Partikeln, wodurch eine Bibliothek erzeugt wird, die im Wesentlichen aus TCRs besteht, die eine Alpha-Kette-variable-Domäne und eine Beta-Kette-variable-Domäne codiert durch die Nukleinsäuren, an die die Primer hybridisieren, umfassen.

13. Verfahren gemäß einem der Ansprüche 11 oder 12, umfassend einen weiteren Schritt, bei dem nicht natürliche Mutationen in die Nukleinsäuren eingeführt werden, gegebenenfalls wobei nicht natürliche Mutationen vor Schritt iii) in die Nukleinsäuren eingeführt werden.

14. Verfahren nach einem der Ansprüche 11 bis 13, wobei die TCR-Alpha-Kette-variable-Domäne und die TCR-Beta-Kette-variable-Domäne TCR als ein Einzelkette-Polypeptid exprimiert werden.

15. Verfahren zur Gewinnung eines T-Zell-Rezeptors, der an ein Peptidantigen spezifisch bindet, umfassend Screening der Bibliothek nach einem der Ansprüche 1 bis 6 mit dem Peptidantigen.

16. Partikel, präsentierend an seiner Oberfläche einen TCR nach Anspruch 7, gegebenenfalls wobei es sich bei dem Partikel um ein Phagenpartikel, ein Ribosom, eine Hefezelle oder eine Säugerzelle handelt.

## Revendications

1. Banque de particules, la banque présentant une pluralité de récepteurs de lymphocyte T (TCR) différents, dans laquelle la pluralité de TCR est essentiellement constituée de TCR comprenant une chaîne alpha comprenant un domaine variable de chaîne alpha provenant d'un répertoire naturel et une chaîne bêta comprenant un domaine variable de chaîne bêta provenant d'un répertoire naturel, dans laquelle le domaine variable de chaîne alpha comprend un produit du gène TRAV12-2 ou TRAV21 et le domaine variable de chaîne bêta comprend un produit du gène TRBV6.

2. Banque de particules, la banque présentant une pluralité de récepteurs de lymphocyte T (TCR) différents, dans laquelle la pluralité de TCR est essentiellement constituée de TCR comprenant une chaîne alpha comprenant un domaine variable de chaîne alpha provenant d'un répertoire naturel et une chaîne bêta comprenant un domaine variable de chaîne bêta provenant d'un répertoire naturel, dans laquelle le domaine variable de chaîne alpha comprend un produit du gène TRAV12-2 ou TRAV21 et le domaine variable de chaîne bêta comprend un produit du gène TRBV6 et dans laquelle au moins une partie des TCR comprennent un domaine variable de chaîne alpha et/ou un domaine variable de chaîne bêta comprenant une mutation non naturelle.

3. Banque selon la revendication 1 ou la revendication 2, dans laquelle le produit du gène TRBV6 est un produit du gène TRBV6-1, TRBV6-2, TRBV6-3, TRBV6-5 ou TRBV6-6.

4. Banque selon l'une quelconque des revendications 1 à 3, dans laquelle le domaine variable de chaîne alpha et le domaine variable de chaîne bêta sont présentés sous la forme d'une chaîne polypeptidique unique.

5. Banque selon l'une quelconque des revendications 1 à 4, dans laquelle les TCR comprennent une liaison disulfure non native ou une liaison disulfure non native entre une région constante de la chaîne alpha et une région constante de la chaîne bêta.

6. Banque selon l'une quelconque des revendications 1 à 5, dans laquelle les particules sont des particules de phage, des ribosomes, des cellules de levure ou des cellules de mammifère.

7. Récepteur de lymphocyte T (TCR) isolé non naturel comprenant un domaine variable de chaîne alpha de TCR comprenant un produit du gène TRAV12-2 ou un produit du gène TRAV21 et un domaine variable de chaîne bêta de TCR comprenant un produit du gène TRBV6 obtenu à partir d'une banque selon la revendication 2.

8. Utilisation d'une banque selon l'une quelconque des revendications 1 à 6 pour identifier un TCR qui se lie spécifiquement à un antigène peptidique.

9. Procédé d'obtention d'un récepteur de lymphocyte T qui se lie spécifiquement à un antigène peptidique, comprenant le criblage de la banque selon la revendication 1 ou la revendication 2 avec l'antigène peptidique, le procédé comprenant :
a) l'adhérence sur plastique de la banque en utilisant, en tant que cible, l'antigène peptidique ;
b) la répétition de l'étape a) une ou plusieurs fois ;
c) le criblage des clones de phage identifiés dans l'étape a) ou b) ; et
d) l'identification d'un TCR qui se lie spécifiquement à l'antigène peptidique.

10. Acide nucléique codant pour un domaine variable de chaîne alpha de TCR et un domaine variable de chaîne bêta du TCR selon la revendication 7.

11. Procédé de fabrication d'une banque de particules, la banque présentant une pluralité de TCR différents, le procédé comprenant :
i) l'obtention d'une pluralité d'acides nucléiques provenant d'un répertoire naturel qui codent pour différents domaines variables de chaîne alpha de TRAV12-2 ou TRAV21 ;
ii) l'obtention d'une pluralité d'acides nucléiques provenant d'un répertoire naturel qui codent pour différents domaines variables de chaîne bêta de TRBV6 ;
iii) le clonage des acides nucléiques codant pour le domaine variable de chaîne alpha TRAV12-2 ou TRAV21 dans des vecteurs d'expression ;
iv) le clonage des acides nucléiques codant pour le domaine variable de chaîne bêta de TRBV6 dans les mêmes vecteurs ou des vecteurs différents ; et
v) l'expression des vecteurs dans des particules, de façon à générer une banque essentiellement constituée de TCR comprenant un domaine variable de chaîne alpha et un domaine variable de chaîne bêta codés par les acides nucléiques.

12. Procédé de fabrication d'une banque de particules, la banque présentant une pluralité de TCR différents, le procédé comprenant :
i) l'obtention d'une pluralité d'acides nucléiques provenant d'un répertoire naturel qui codent pour différents domaines variables de chaîne alpha de TRAV12-2 ou TRAV21 en utilisant des amorces qui s'hybrident à des acides nucléiques codant pour les domaines variables de chaîne alpha de TRA12-2 ou TRAV21 ;
ii) l'obtention d'une pluralité d'acides nucléiques provenant d'un répertoire naturel qui codent pour différents domaines variables de chaîne bêta de TRBV6 en utilisant des amorces qui s'hybrident à des acides nucléiques codant pour les domaines variables de chaîne TRAV6 ;
iii) le clonage des acides nucléiques codant pour le domaine variable de chaîne alpha TRAV12-2 ou TRAV21 dans des vecteurs d'expression ;
iv) le clonage des acides nucléiques codant pour le domaine variable de chaîne bêta de TRBV6 dans les mêmes vecteurs ou des vecteurs différents ; et
v) l'expression des vecteurs dans des particules, de façon à générer une banque essentiellement constituée de TCR comprenant un domaine variable de chaîne alpha et un domaine variable de chaîne bêta codés par les acides nucléiques auxquels lesdites amorces s'hybrident.

13. Procédé selon l'une quelconque des revendications 11 ou 12, comprenant une étape supplémentaire d'introduction de mutations non naturelles dans les acides nucléiques, facultativement dans lequel les mutations non naturelles sont introduites dans les acides nucléiques avant l'étape iii).

14. Procédé selon l'une quelconque des revendications 11 à 13, dans lequel le domaine variable de chaîne alpha de TCR et le domaine variable de chaîne bêta de TCR sont exprimés sous la forme d'un polypeptide monocaténaire.

15. Procédé d'obtention d'un récepteur de lymphocyte T qui se lie spécifiquement à un antigène peptidique, comprenant le criblage de la banque selon l'une quelconque des revendications 1 à 6 avec l'antigène peptidique.

16. Particule présentant sur sa surface un TCR selon la revendication 7, la particule étant facultativement une particule de phage, un ribosome, une cellule de levure ou une cellule de mammifère.
